# EUROPEAN PATENT APPLICATION

(11) **EP 3 549 610 A1**
(43) Date of publication of application: **09.10.2019**
(21) Application number: 18165986.3
(22) Date of filing: 05.04.2018
(51) Int. Cl.: A61K 47/54

(54) **NUCLEIC ACID CONJUGATES**

(71) Applicant: Silence Therapeutics GmbH, 13125 Berlin (DE)
(72) Inventor: The designation of the inventor has not yet been filed
(74) Representative: Teuten, Andrew John

(57) **Abstract**

There is provided *inter alia* a conjugate for inhibiting expression of a target gene in a cell, said conjugate comprising a nucleic acid portion and ligand portions, said nucleic acid portion comprising at least one duplex region that comprises at least a portion of a first RNA strand and at least a portion of a second RNA strand that is at least partially complementary to the first strand, wherein said first strand is at least partially complementary to at least a portion of RNA transcribed from said target gene, said ligand portions comprising a serinol-derived linker moiety and a targeting ligand for *in vivo* targeting of cells and being conjugated exclusively to the 3' and/or 5' ends of one or both RNA strands, wherein the 5' end of the first RNA strand is not conjugated, wherein:
(i) the second RNA strand is conjugated at the 5' end to the targeting ligand, and wherein (a) the second RNA strand is also conjugated at the 3' end to the targeting ligand and the 3' end of the first RNA strand is not conjugated; or (b) the first RNA strand is conjugated at the 3' end to the targeting ligand and the 3' end of the second RNA strand is not conjugated; or (c) both the second RNA strand and the first RNA strand are also conjugated at the 3' ends to the targeting ligand; or
(ii) both the second RNA strand and the first RNA strand are conjugated at the 3' ends to the targeting ligand and the 5' end of the second RNA strand is not conjugated.

## Description

### Technical Field

The present invention relates to novel nucleic acid conjugate compounds. The invention further relates to compositions comprising said conjugates and their use in medicine, research and diagnostics. The novel conjugate compounds may be used in the treatment of many diseases including central-nervous-system diseases, inflammatory diseases, metabolic disorders, genetic and inherited diseases, oncology, infectious diseases, and ocular disease.

### Background

Double-stranded RNA (dsRNA) has been shown to block gene expression (Fire et al., 1998 and Elbashir et al., 2001) and this has been termed RNA interference or "RNAi", mediated by interfering RNA molecules (RNAi). Short dsRNA directs gene-specific, post-transcriptional silencing in many organisms, including vertebrates, and has provided a new tool for studying gene function. RNAi is mediated by RNA-induced silencing complex (RISC), a sequence-specific, multi-component nuclease that destroys messenger RNAs homologous to the silencing trigger. iRNAs (interfering RNA) such as siRNA (short interfering RNA), antisense RNA, and micro-RNA are oligonucleotides that prevent the formation of proteins by gene-silencing i.e. inhibiting translation of the protein. Gene-silencing agents are becoming increasingly important for therapeutic applications in medicine.

Thus, means for efficient delivery of oligonucleotides, in particular double stranded siRNAs, to cells *in vivo* are becoming increasingly important and require specific targeting and substantial protection from the extracellular environment, particularly serum proteins. One method of achieving specific targeting is to conjugate a targeting moiety to the RNAi duplex agent. The targeting moiety helps in targeting the RNAi duplex agent to the required target site and there is a need to design appropriate targeting moieties for the desired receptor sites for the conjugated molecules to be taken up by the cells such as by endocytosis.

For example, the asialoglycoprotein receptor (ASGPR) is a high capacity receptor, which is highly abundant on hepatocytes and it has been known for more than ten years that GalNAc-siRNA conjugates are sufficient to target and deliver siRNA into hepatocytes *in vivo.* While in the past, trimeric clusters were preferred due to higher binding affinity to the asialogylcoprotein receptor (ASGPR) some more recent publications report on two single GalNAc moieties to be sufficient.

Matsuda *et al.* (2015) describes conjugates which have conjugation of the RNA strands to GalNAc within the RNA strands.

Schmidt *et al.* (2017) describes one, two or three-GalNAc conjugated single stranded and double stranded DNA antisense oligonucleotides (ASOs).

Kamiya *et al.* (2014) discloses a study which investigates terminal substitution of siRNAs.

However, targeting ligands developed so far do not always translate to *in vivo* setting and there is a clear need for more efficacious receptor specific ligand conjugated RNAi duplex agents and methods for their preparation for the *in vivo* delivery of oligonucleotide therapeutics, nucleic acids and double stranded siRNAs. The present invention attempts to address these needs.

The present invention relates to the finding that nucleic acid conjugates of particular structures are potent with long duration of action and have surprising improved *in vivo* activity over other nucleic acid conjugates.

### Summary of the invention

The present invention relates to a conjugate for inhibiting expression of a target gene in a cell, said conjugate comprising a nucleic acid portion and ligand portions, said nucleic acid portion comprising at least one duplex region that comprises at least a portion of a first RNA strand and at least a portion of a second RNA strand that is at least partially complementary to the first strand, wherein said first strand is at least partially complementary to at least a portion of RNA transcribed from said target gene, said ligand portions comprising a serinol-derived linker moiety and a targeting ligand for *in vivo* targeting of cells and being conjugated exclusively to the 3' and/or 5' ends of one or both RNA strands, wherein the 5' end of the first RNA strand is not conjugated, wherein:
(i) the second RNA strand is conjugated at the 5' end to the targeting ligand, and wherein (a) the second RNA strand is also conjugated at the 3' end to the targeting ligand and the 3' end of the first RNA strand is not conjugated; or (b) the first RNA strand is conjugated at the 3' end to the targeting ligand and the 3' end of the second RNA strand is not conjugated; or (c) both the second RNA strand and the first RNA strand are also conjugated at the 3' ends to the targeting ligand; or
(ii) both the second RNA strand and the first RNA strand are conjugated at the 3' ends to the targeting ligand and the 5' end of the second RNA strand is not conjugated.

As used herein, the first strand may be referred to as the antisense strand and the second strand may be referred to as the sense strand. The terms first strand and antisense strand or second strand and sense strand should be treated as interchangeable.

In the various aspects of the invention (unless the stated otherwise) the targeting ligand may be any targeting ligand appropriate for the cell to be targeted. In one preferred embodiment, the targeting ligand targets ASGP receptors, especially such receptors on liver cells. For example, the targeting ligand is or comprises a saccharide moiety such as galactose, mannose, glucose, glucosamine, fucose and fructose or derivatives thereof such as N-acetyl derivatives thereof e.g. GalNAc. The preferred targeting ligand is GalNAc.

### Brief description of Figures

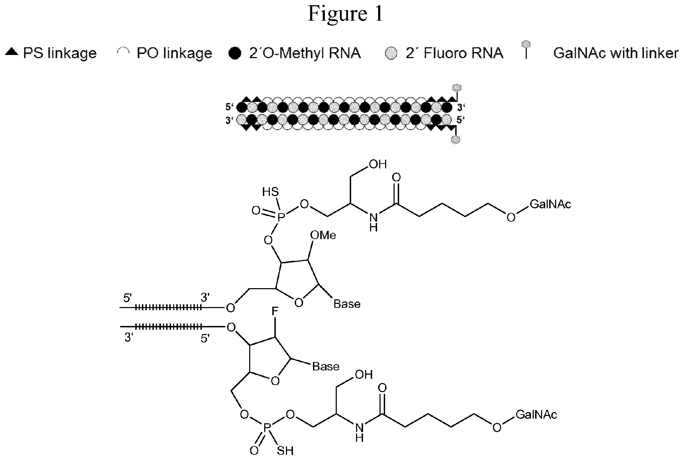

**Figure 1** depicts Conjugate 1. The last three nucleotides at the 5' and 3' ends of the antisense and sense strands are connected by a phosphorothioate linker between each nucleotide. The serinol-GalNAc-linkers are conjugated via a phosphorothioate bond to the 3' end of the antisense strand as well as to the 5' end of the sense strand.

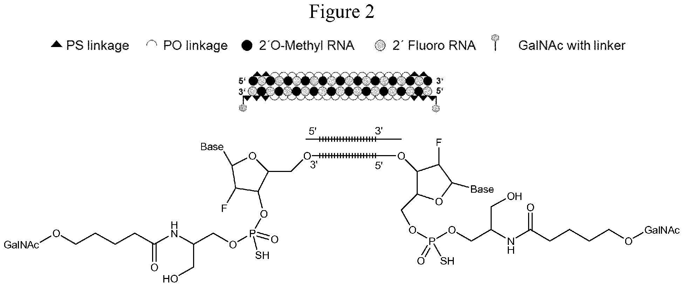

**Figure 2** depicts Conjugate 2. The last three nucleotides at the 5' and 3' ends of the antisense and sense strands are connected by a phosphorothioate linker between each nucleotide. The serinol-GalNAc-linkers are conjugated via a phosphorothioate bond to the 3' end and the 5' end of the sense strand.

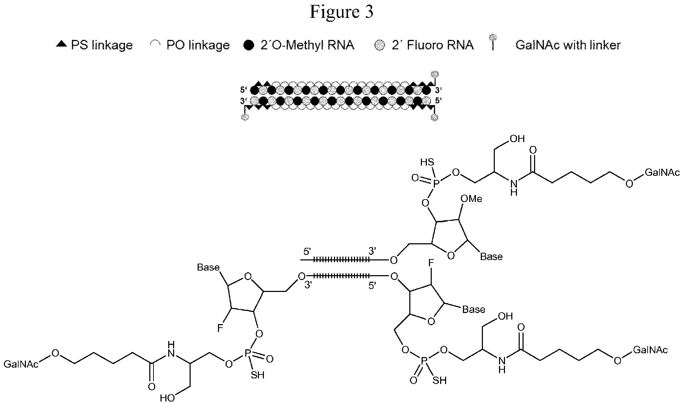

**Figure 3** depicts Conjugate 3. The last three nucleotides at the 5' and 3' ends of the antisense and sense strands are connected by a phosphorothioate linker between each nucleotide. The serinol-GalNAc-linkers are conjugated via a phosphorothioate bond to the 3' end and the 5' end of the sense strand as well as to the 3' end of the antisense strand.

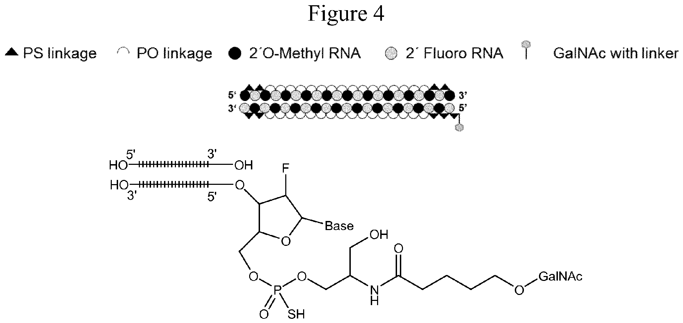

**Figure 4** depicts Reference Conjugate 1. The last three nucleotides at the 5' and 3' ends of the antisense and sense strands are connected by a phosphorothioate linker between each nucleotide. The serinol-GalNAc-linkers are conjugated via a phosphorothioate bond to the 5' end of the sense strand.

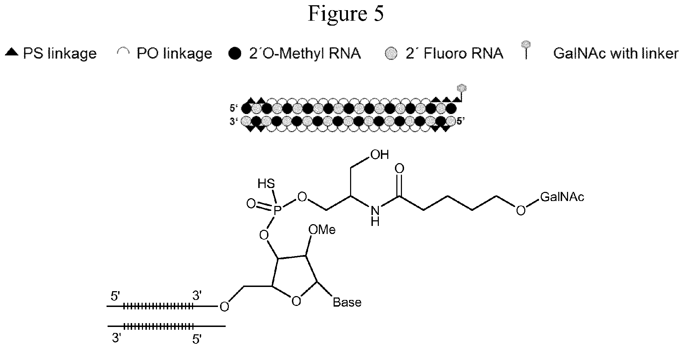

**Figure 5** depicts Reference Conjugate 2. The last three nucleotides at the 5' and 3' ends of the antisense and sense strands are connected by a phosphorothioate linker between each nucleotide. The serinol-GalNAc-linkers are conjugated via a phosphorothioate bond to the 3' end of the antisense strand.

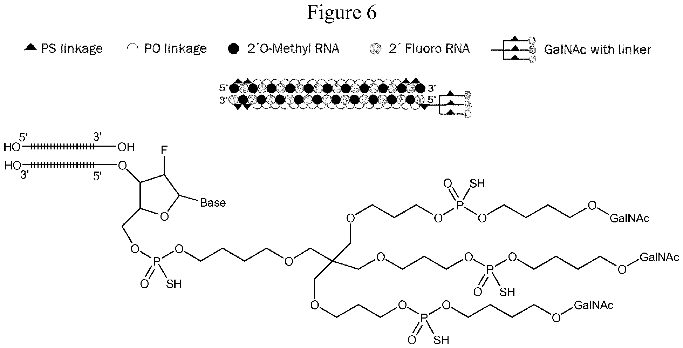

**Figure 6** depicts Reference Conjugate 3. The last three nucleotides at the 5' and 3' ends of the antisense and the 3' end of the sense strands are connected by a phosphorothioate linker between each nucleotide. The trimeric GalNAc-linker is conjugated via a phosphorothioate bond to the 5' end of the sense strand.

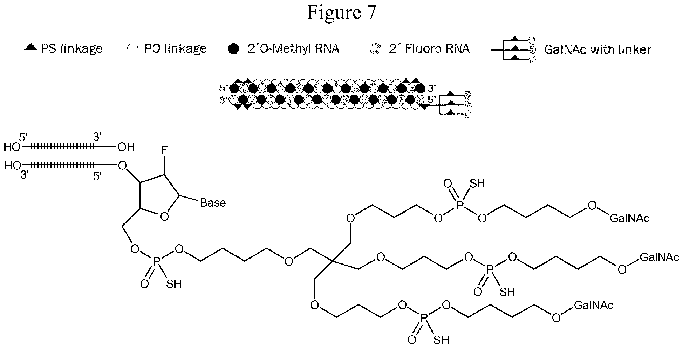

**Figure 7** depicts Reference Conjugate 4. The last three nucleotides at the 5' and 3' ends of the antisense and the 3' end of the sense strands are connected by a phosphorothioate linker between each nucleotide. The trimeric GalNAc-linker is conjugated via a phosphorothioate bond to the 5' end of the sense strand.
   In each of Figures 1-7, the top strand is the antisense strand and the bottom strand is the sense strand i.e. 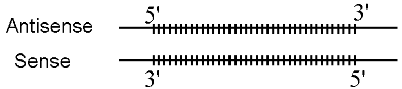
Antisense 5' 3'
Sense 3' 5'
 In addition, to show more clearly the connection between the nucleic acid and ligand portions, the nucleotide at the end of the respective conjugated strands is drawn in full.

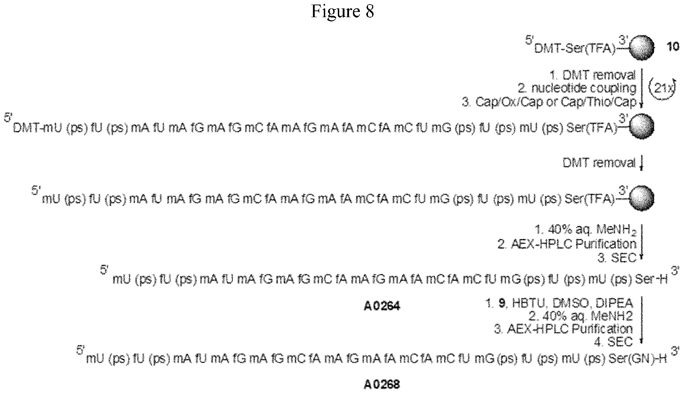

**Figure 8** shows the synthesis of **A0268** which is a 3' mono-GalNAc conjugated single stranded oligonucleotide and is the starting material in the synthesis of Conjugate 1 and Conjugate 3. (ps) denotes phosphorothioate linkage.

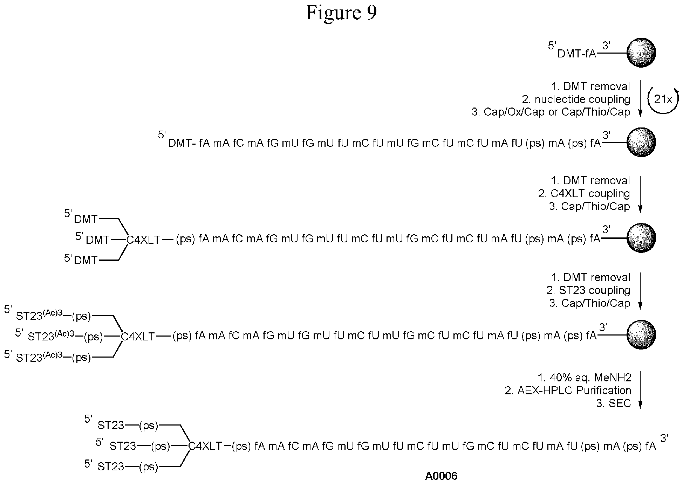

**Figure 9** shows the synthesis of **A0006** which is a 5' tri-antennary GalNAc conjugated single stranded oligonucleotide used for the synthesis of Reference Conjugate 4. (ps) denotes phosphorothioate linkage.

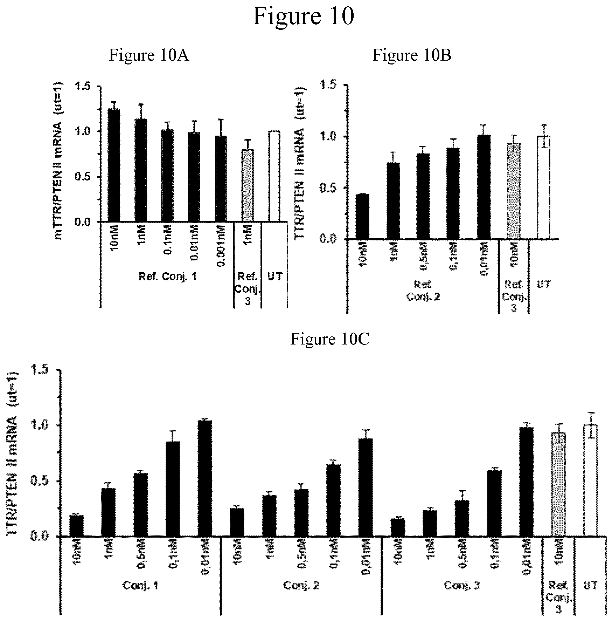

**Figure 10** illustrates the *in vitro* determination of TTR knockdown. In particular, **Figure 10A** shows the *in vitro* determination of TTR knockdown by Reference Conjugates (RC) 1 and 3 as well as the untreated control "UT"; **Figure 10B** shows the *in vitro* determination of TTR knockdown by Reference Conjugates (RC) 2 and 3, as well as by the untreated control "UT"; and **Figure 10C** shows the *in vitro* determination of TTR knockdown by Conjugates 1, 2 and 3, as well as by RC3 and untreated control "UT". Reference Conjugates 1 and 2 represent comparator conjugates. Reference Conjugate 3 represents a non-targeting GalNAc siRNA and "untreated" ("UT") represents untreated cells. Both RC3 and UT are negative controls. mRNA level were normalised against Ptenll.

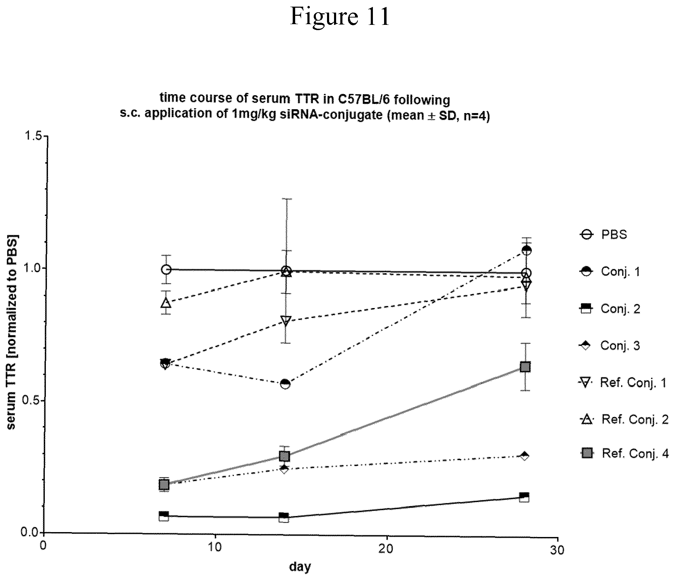

**Figure 11** shows a time course of serum TTR in c57BL/6 mice cohorts of n=4 at 7, 14, and 27 days post s.c. treatment with 1mg/kg - Conjugates 1-3, Reference Conjugates (RC) 1, 2 and 4 and mock treated (PBS) individuals.

### Detailed description of the invention

The definitions and explanations below are for the terms as used throughout this entire document including both the specification and the claims.

Unless specified otherwise, the following terms have the following meanings: **"GalNAc"** means N-acetyl galactosamine which is also known as 2-(Acetylamino)-2-deoxy-D-galactopyranose. Reference to "GalNAc" or "N-acetyl galactosamine" includes both the beta-form: 2-(Acetylamino)-2-deoxy-beta-D-galactopyranose and the alpha-form: 2-(Acetylamino)-2-deoxy-alpha-D-galactopyranose. In certain embodiments, both the beta-form: 2-(Acetylamino)-2-deoxy-beta-D-galactopyranose and alpha-form: 2-(Acetylamino)-2-deoxy-alpha-D-galactopyranose may be used interchangeably. Preferably, the compounds of the invention comprise the beta-form, 2-(Acetylamino)-2-deoxy-beta-D-galactopyranose. 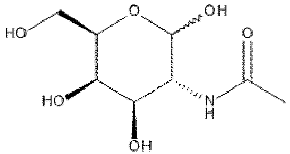 2-(Acetylamino)-2-deoxy-D-galactopyranose 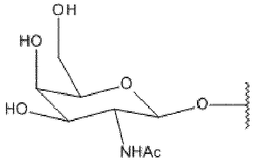 2-(Acetylamino)-2-deoxy-beta-D-galactopyranose 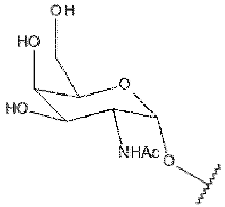 2-(Acetylamino)-2-deoxy-alpha-D-galactopyranose

The term **"C₁-C₁₅ alkyl"** refers to a saturated aliphatic hydrocarbon group having 1-15 carbon atoms which may be linear or branched. For example C₁-C₆ alkyl and includes C₁ (CH₃), C₂ (CH₂CH₃), C₃ ((CH₂)₂CH₃), C₄ ((CH₂)₃CH₃), C₅ ((CH₂)₄CH₃) and C₆ ((CH₂)₅CH₃). "Branched" means that at least one carbon branch point is present in the group. For example, tert-butyl and isopropyl are both branched groups. Examples of C₁-C₆ alkyl groups include methyl, ethyl, propyl, butyl, 2-methyl-1-propyl, 2-methyl-2-propyl, 2-methyl-1-butyl, 3 methyl-1-butyl, 2-methyl-3-butyl, 2,2-dimethyl-1-propyl, 2-methyl-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, n-butyl, isobutyl, tert-butyl, n-pentyl, isopentyl, neopentyl and n-hexyl. This also applies for C₁-C₆ alkylene which has the meaning as follows: C₁ (CH₂), C₂ (CH₂CH₂), C₃ ((CH₂)₃), C₄ ((CH₂)₄), C₅ ((CH₂)₅) and C₆ ((CH₂)₆).

The term **"conjugated exclusively to the 3' and/or 5' ends"** means that the ligand may only be conjugated to the 3' ends and/or the 5' ends of one or both RNA strands, and excludes the possibility for the ligand to be conjugated to the oligonucleotide chain at any other location e.g. to a base.

The term **"ligand"** or **"targeting ligand"** refers to a moiety (or several moieties) such as a saccharide, such as a galactosamine derivative e.g. GalNAc which may be selected to have an affinity for at least one type of receptor on a target cell. In particular, the receptor is on the surface of a mammalian liver cell, for example, the hepatic asialoglycoprotein receptor (ASGPR).

The term **"monomeric ligand"** means a ligand comprising only a single moiety which has affinity for at least one type of receptor on a target cell e.g. a single monosaccharide e.g. a single galactosamine derivative (e.g. GalNAc) moiety.

The term **"nucleic acid"** refers to RNA which forms a double stranded RNA, in particular, siRNA.

The term **"serinol-derived linker moiety"** means the linker moiety comprises the following structure:

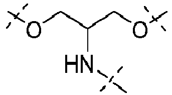

An O atom of said structure typically links to an RNA strand and the N atom typically links to the targeting ligand.

The moiety may comprise other groups such as methyl groups, such as a methyl group, for example a methyl group in the alpha-position:

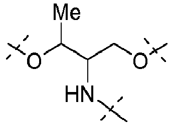

The moiety may comprise a further linker group such as group L defined below, interposed between the N atom of the serinol-derived linker moiety and the targeting ligand. A further linker may also be present interposed between an O atom of the serinol-derived linker moiety and the RNA strand.

The term **"treat"** or **"treating"** or **"treatment"** may include prophylaxis and means to ameliorate, alleviate symptoms, eliminate the causation of the symptoms either on a temporary or permanent basis, or to prevent or slow the appearance of symptoms of the named disorder or condition. The compounds of the invention are useful in the treatment of humans and non-human animals.

By **"effective amount"** or **"therapeutically effective amount"** or **"effective dose"** is meant that amount sufficient to elicit the desired pharmacological or therapeutic effects, thus resulting in effective prevention or treatment of the disorder. Prevention of the disorder is manifested by delaying the onset of the symptoms of the disorder to a medically significant extent. Treatment of the disorder is manifested by a decrease in the symptoms associated with the disorder or an amelioration of the reoccurrence of the symptoms of the disorder.

A **"pharmaceutical composition"** or **"composition"** means a mixture of substances suitable for administering to an individual. For example, a pharmaceutical composition can comprise one or more active agents and a pharmaceutical carrier e.g. a sterile aqueous solution.

The term **"alternating"** as described herein means to occur one after another in a regular way. In other words, alternating means to occur in turn repeatedly. For example if one nucleotide is modified, the next contiguous nucleotide is not modified and the following contiguous nucleotide is modified and so on. One nucleotide may be modified with a first modification, the next contiguous nucleotide may be modified with a second modification and the following contiguous nucleotide is modified with the first modification and so on, where the first and second modifications are different.

The term **"inhibit", "down-regulate",** or **"reduce"** with respect to gene expression means the expression of the gene, or level of RNA molecules or equivalent RNA molecules encoding one or more proteins or protein subunits (e.g., mRNA), or activity of one or more proteins or protein subunits, is reduced below that observed in the absence of a nucleic acid of the invention; for example the expression may be reduced to 90%, 80%, 70%, 60%, 50%, 40%, 30%, 20%, 10%, 5% or less than that observed in the absence of an inhibitor.

The term **"first RNA strand"** as used herein means the antisense strand, and are used interchangeably throughout the application.

The term **"second RNA strand"** as used herein means the sense strand, and are used interchangeably throughout the application.

The present invention relates to a conjugate for inhibiting expression of a target gene in a cell, said conjugate comprising a nucleic acid portion and ligand portions, said nucleic acid portion comprising at least one duplex region that comprises at least a portion of a first RNA strand and at least a portion of a second RNA strand that is at least partially complementary to the first strand, wherein said first strand is at least partially complementary to at least a portion of RNA transcribed from said target gene, said ligand portions comprising a serinol-derived linker moiety and a targeting ligand for *in vivo* targeting of cells and being conjugated exclusively to the 3' and/or 5' ends of one or both RNA strands, wherein the 5' end of the first RNA strand is not conjugated, wherein:
(i) the second RNA strand is conjugated at the 5' end to the targeting ligand, and wherein (a) the second RNA strand is also conjugated at the 3' end to the targeting ligand and the 3' end of the first RNA strand is not conjugated; or (b) the first RNA strand is conjugated at the 3' end to the targeting ligand and the 3' end of the second RNA strand is not conjugated; or (c) both the second RNA strand and the first RNA strand are also conjugated at the 3' ends; or
(ii) both the second RNA strand and the first RNA strand are conjugated at the 3' ends and the 5' end of the second RNA strand is not conjugated.

In an embodiment of the present invention, the second RNA strand (i.e. the sense strand) is conjugated at the 5' end to the targeting ligand, the first RNA strand (i.e. the antisense strand) is conjugated at the 3' end to the targeting ligand and the 3' end of the second RNA strand (i.e. the sense strand) is not conjugated, such that a conjugate with the following schematic structure is formed:

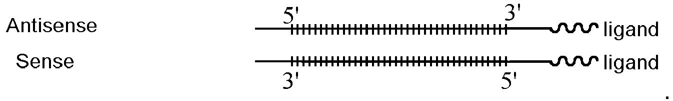
Antisense 5' 3' ligand
Sense 3' 5' ligand
.

In an embodiment of the present invention, the second RNA strand (i.e. the sense strand) is conjugated at the 5' end to the targeting ligand, the second RNA strand (i.e. the sense strand) is also conjugated at the 3' end to the targeting ligand and the 3' end of the first RNA strand (i.e. the antisense strand) is not conjugated, such that a conjugate with the following schematic structure is formed:

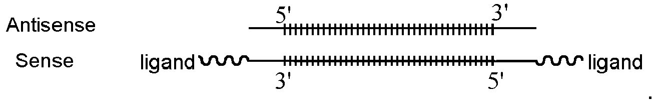
Antisense 5' 3'
Sense ligand 3' 5' ligand
.

In an embodiment of the present invention, both the second RNA strand (i.e. the sense strand) and the first RNA strand (i.e. the antisense strand) are conjugated at the 3' ends to the targeting ligand and the 5' end of the second RNA strand (i.e. the sense strand) is not conjugated , such that a conjugate with the following schematic structure is formed:

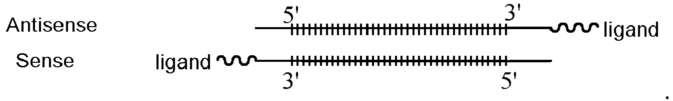
Antisense 5' 3' ligand
Sense ligand 3' 5'
.

In an embodiment of the present invention, the second RNA strand (i.e. the sense strand) is conjugated at the 5' end to the targeting ligand and both the second RNA strand (i.e. the sense strand) and the first RNA strand (i.e. the antisense strand) are also conjugated at the 3' ends to the targeting ligand , such that a conjugate with the following schematic structure is formed:

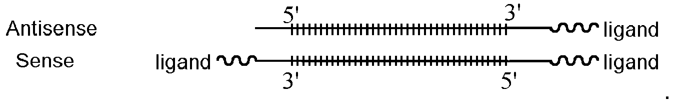
Antisense 5' 3' ligand
Sense ligand 3' 5' ligand
.

In any one of the above embodiments, 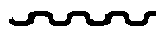 indicates the linker which conjugates the ligand to the ends of the nucleic acid portion; the ligand may be a GalNAc moiety such as GalNAc; and wherein 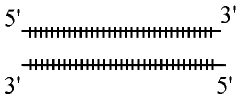 represents the nucleic acid portion.

These schematic diagrams are not intended to limit the number of nucleotides in the first or second strand, nor do the diagrams represent any kind of limitation on complementarity of the bases or any other limitation.

The ligands may be monomeric or multimeric (e.g. dimeric, trimeric, etc.).

Suitably, the ligands are monomeric, thus containing a single targeting ligand moiety, e.g. a single GalNAc moiety.

Alternatively, the ligands may be dimeric ligands wherein the ligand portions comprise two serinol-derived linker moieties, each linked to a single targeting ligand moiety.

The ligands may be trimeric ligands wherein the ligand portions comprise three serinol-derived linker moieties, each linked to a single targeting ligand moiety.

The two or three serinol-derived linker moieties may be linked in series e.g. as shown below: 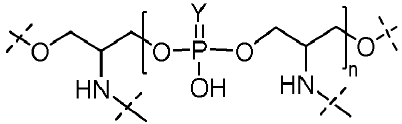 wherein n is 1 or 2 and Y is S or O.

Preferably, the ligands are monomeric.

Suitably, the conjugated RNA strands are conjugated to a targeting ligand via a serinol-derived linker moiety including a further linker wherein the further linker is or comprises a saturated, unbranched or branched C₁₋₁₅ alkyl chain, wherein optionally one or more carbons (for example 1, 2 or 3 carbons, suitably 1 or 2, in particular 1) is/are replaced by a heteroatom selected from O, N, S(O)ₚ, wherein p is 0, 1 or 2 (for example a CH₂ group is replaced with O, or with NH, or with S, or with SO₂ or a -CH₃ group at the terminus of the chain or on a branch is replaced with OH or with NH₂) wherein said chain is optionally substituted by one or more oxo groups (for example 1 to 3, such as 1 group).

More suitably, the further linker comprises a saturated, unbranched C₁₋₁₅ alkyl chain wherein one or more carbons (for example 1, 2 or 3 carbons, suitably 1 or 2, in particular 1) is/are replaced by an oxygen atom.

More suitably, the further linker comprises a PEG-chain.

More suitably, the further linker comprises a saturated, unbranched C₁₋₁₅ alkyl chain.

More suitably, the further linker comprises a saturated, unbranched C₁₋₆ alkyl chain.

More suitably, the further linker comprises a saturated, unbranched C₄ or C₆ alkyl chain, e.g. a C₄ alkyl chain.

In an embodiment, 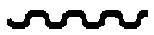 is a linking moiety of formula (III): 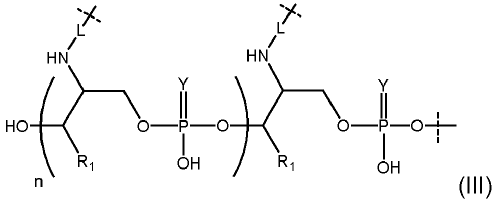 wherein n, Y, R₁ and L are defined below, L is connected to the targeting ligand e.g. GalNAc and the O of the phosphoro-group is attached to the terminal oligonucleoside of the RNA strands.

Suitably, the targeting ligand portion is a linking moiety of formula (IV): 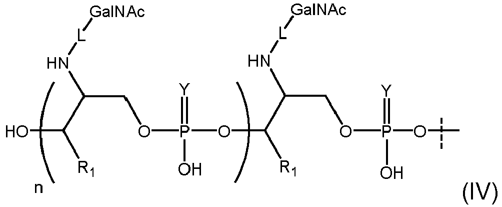 wherein n, Y, R₁ and L are defined below and the O of the phosphoro-group is attached to the terminal oligonucleoside of the RNA strands.

Suitably, L is:

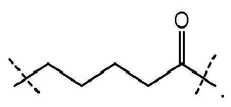

In any of the above structures, suitably the ligands are selected from GalNAc and galactose moieties, especially GalNAc moieties. Alternatively, GalNac may be replaced by another targeting ligand, e.g. a saccharide.

In an embodiment of the invention, the first RNA strand is a compound of formula (I): 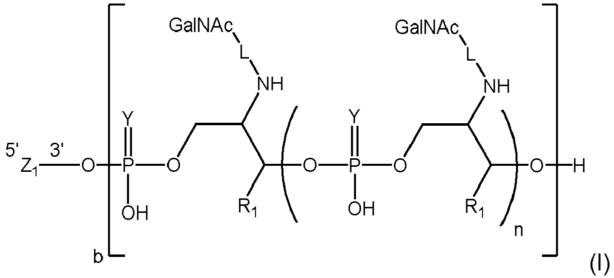 wherein b is 0 or 1; and
the second RNA strand is a compound of formula (II): 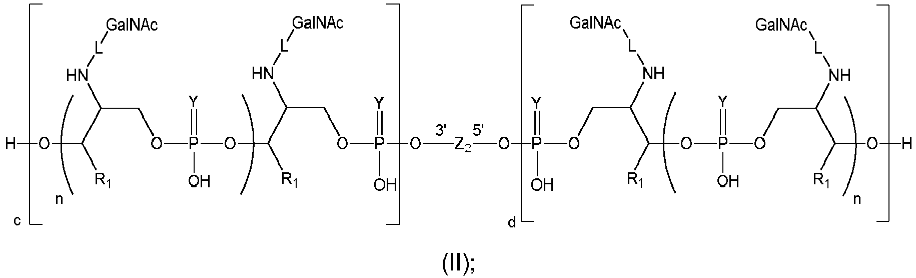 wherein c and d are independently 0 or 1; wherein:
Z₁ and Z₂ are the RNA portions of the first and second RNA strands respectively;
Y is O or S;
R₁ is H or methyl;
n is 0, 1, 2 or 3; and
L is the same or different in formulae (I) and (II) and is selected from the group consisting of:
   - (CH₂)ᵣ-C(O)-, wherein r = 2-12;
   - (CH₂-CH₂-O)ₛ-CH₂-C(O)-, wherein s = 1-5;
   - (CH₂)ₜ-CO-NH-(CH₂)ₜ-NH-C(O)-, wherein t is independently is 1-5;
   - (CH₂)ᵤ-CO-NH-(CH₂)ᵤ-C(O)-, wherein u is independently is 1-5; and
   - (CH₂)ᵥ-NH-C(O)-, wherein v is 2-12; and
wherein the terminal C(O) (if present) is attached to the NH group;
and wherein b + c + d is 2 or 3.

Suitably, b is 0, c is 1 and d is 1; b is 1, c is 0 and d is 1; b is 1, c is 1 and d is 0; or b is 1, c is 1 and d is 1.

More suitably, b is 0, c is 1 and d is 1; b is 1, c is 0 and d is 1; or b is 1, c is 1 and d is 1.

Most suitably, b is 0, c is 1 and d is 1.

In one embodiment, Y is O. In another embodiment, Y is S.

In one embodiment, R₁ is H or methyl. In one embodiment, R₁ is H. In another embodiment, R₁ is methyl.

In one embodiment, n is 0, 1, 2 or 3. Suitably, n is 0.

In one embodiment, L is selected from the group consisting of:
- (CH₂)ᵣ-C(O)-, wherein r = 2-12;
- (CH₂-CH₂-O)ₛ-CH₂-C(O)-, wherein s = 1-5;
- (CH₂)ₜ-CO-NH-(CH₂)ₜ-NH-C(O)-, wherein t is independently is 1-5;
- (CH₂)ᵤ-CO-NH-(CH₂)ᵤ-C(O)-, wherein u is independently is 1-5; and
- (CH₂)ᵥ-NH-C(O)-, wherein v is 2-12;
wherein the terminal C(O) is attached to the NH group.

Suitably, L is -(CH₂)ᵣ-C(O)-, wherein r = 2-12. Suitably, r = 2-6. More suitably, r = 4 or 6 e.g. 4.

Serinol derived linker moieties may be based on serinol in any stereochemistry i.e. derived from L-serine isomer, D-serine isomer, a racemic serine or other combination of isomers. In a preferred aspect of the invention, the serinol-GalNAc moiety (SerGN) has the following stereochemistry: 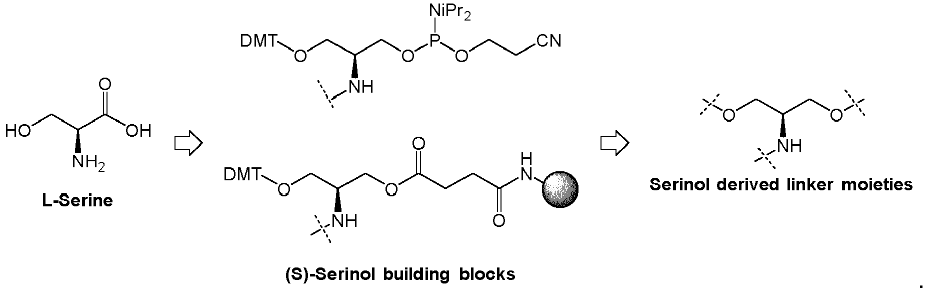 i.e. is based on an (S)-serinol-amidite or (S)-serinol succinate solid supported building block derived from L-serine isomer.

In one embodiment, the targeted cells are hepatocytes.

### General synthesis schemes

Example compounds can be synthesised according to methods described below and known to the person skilled in the art. Assembly of the oligonucleotide chain and linker building blocks may, for example, be performed by solid phase synthesis applying phosphoramidte methodology. Solid phase synthesis may start from a base or modified building block loaded Icaa CPG. Phosphoramidite synthesis coupling cycle consists of 1) DMT-removal, 2) chain elongation using the required DMT-masked phosphoramidite and an activator, which may be benzylthiotetrazole (BTT), 3) capping of non-elongated oligonucleotide chains, followed by oxidation of the P(III) to P(V) either by Iodine (if phosphodiester linkage is desired) or EDITH (if phosphorothioate linkage is desired) and again capping (Cap/Ox/Cap or Cap/Thio/Cap). GalNAc conjugation may be achieved by peptide bond formation of a GalNAc-carboxylic acid building block to the prior assembled and purified oligonucleotide having the necessary number of amino modified linker building blocks attached. The necessary building blocks are either commercially available or synthesis is described below. All final single stranded products were analysed by AEX-HPLC to prove their purity. Purity is given in %FLP (% full length product) which is the percentage of the UV-area under the assigned product signal in the UV-trace of the AEX-HPLC analysis of the final product. Identity of the respective single stranded products was proved by LC-MS analysis.

### Synthesis of Synthons

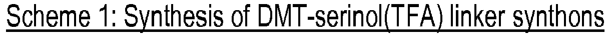

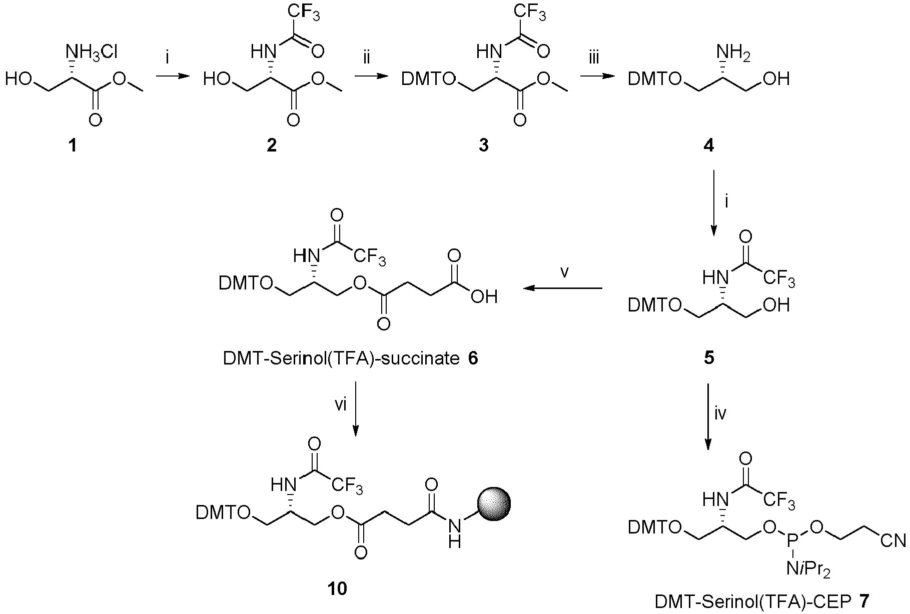

i) ethyl trifluoroacetate, NEt₃, MeOH, 0°C, 16h, **2:** 86% **5:** 90%, ii) DMTCI, pyridine, 0°C, 16h, 74%, iii) LiBH4, EtOH/THF (1/1, v/v), 0°C, 1h, 76%, iv) 2-cyanoethyl-N,N-diisopropylchloro phosphoramidite, EtN*i*Pr₂, CH₂Cl₂, 56%, v) succinic anhydride, DMAP, pyridine, RT, 16h, 38%, vi) HBTU, DIEA, amino-lcaa CPG (500A), RT, 18h, 29% (26 umol/g loading).

(S)-DMT-Serinol(TFA)-phosphoramidite **7** can be synthesised from (L)-serine methyl ester derivative **1** according to literature published methods (Hoevelmann et al. Chem. Sci., 2016,7, 128-135).

(S)-DMT-Serinol(TFA)-succinate **6** can be made by conversion of intermediate **5** with succinic anhydride in presence of a catalyst such as DMAP.

Loading of **6** to a solid support such as a controlled pore glass (CPG) support may be achieved by peptide bond formation to a solid support such as an amino modified native CPG support (500A) using a coupling reagent such as HBTU. The (S)-DMT-Serinol(TFA)-succinate **6** and a coupling reagent such as HBTU is dissolved in a solvent such as CH₃CN. A base, such as diisopropylethylamine, is added to the solution, and the reaction mixture is stirred for 2 min. A solid support such as a native amino-lcaa-CPG support (500 A, 3 g, amine content: 136 umol/g) is added to the reaction mixture and a suspension forms. The suspension is gently shaken at room temperature on a wrist-action shaker for 16h then filtered, and washed with solvent such as DCM and EtOH. The support is dried under vacuum for 2 h. The unreacted amines on the support can be capped by stirring with acetic anhydride/lutidine/N-methylimidazole at room temperature. Washing of the support may be repeated as above. The solid support is dried under vacuum to yield solid support **10.**

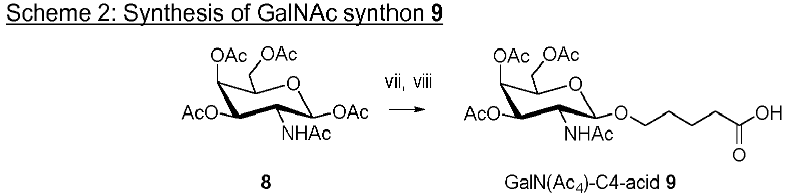

(vii) TMSOTf, DCM, hexenol, viii) RuCl₃, NalO₄, DCM, CH₃CN, H₂O, 46% over two steps. Synthesis of the GalNAc synthon **9** can be prepared according to methods as described in Nair et al. (2014), starting from commercially available per-acetylated galactose amine **8.**

### Synthesis of the single stranded serinol-derived GalNAc conjugates in conjugates 1-3 and reference conjugates 1-2

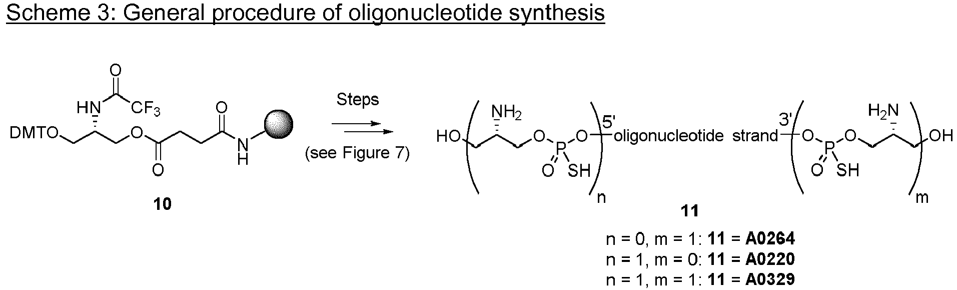

Oligonucleotide synthesis of 3' mono-GalNAc conjugated oligonucleotides (such as compound **A0264**) is outlined in Figure 8 and summarised in Scheme 3. Synthesis is commenced using (S)-DMT-Serinol(TFA) -succinate-lcaa-CPG **10** as in example compound **A0264.** In case additional serinol building blocks are needed the (S)-DMT-serinol(TFA) amidite (**7**) is used in the appropriate solid phase synthesis cycle. For example, to make compound **A0329,** the chain assembly is finished with an additional serinol amidite coupling after the base sequence is fully assembled. Further, oligonucleotide synthesis of 5' mono-GalNAc conjugated oligonucleotides may be commenced from a solid support loaded with the appropriate nucleoside of its respected sequence. In example compound **A0220** this may be 2'fA. The oligonucleotide chain is assembled according to its sequence and as appropriate, the building block (S)-DMT-serinol(TFA)-amidite (**7**) is used. Upon completion of chain elongation, the protective DMT group of the last coupled amidite building block is removed, as in step 1) of the phosphoramidite synthesis cycle.

Upon completion of the last synthesizer step, the single strands can be cleaved off the solid support by treatment with an amine such as 40% aq. methylamine treatment. Any remaining protecting groups are also removed in this step and methylamine treatment also liberates the serinol amino function. The crude products were then purified each by AEX-HPLC and SEC to yield the precursor oligonucleotide for further GalNAc conjugation.

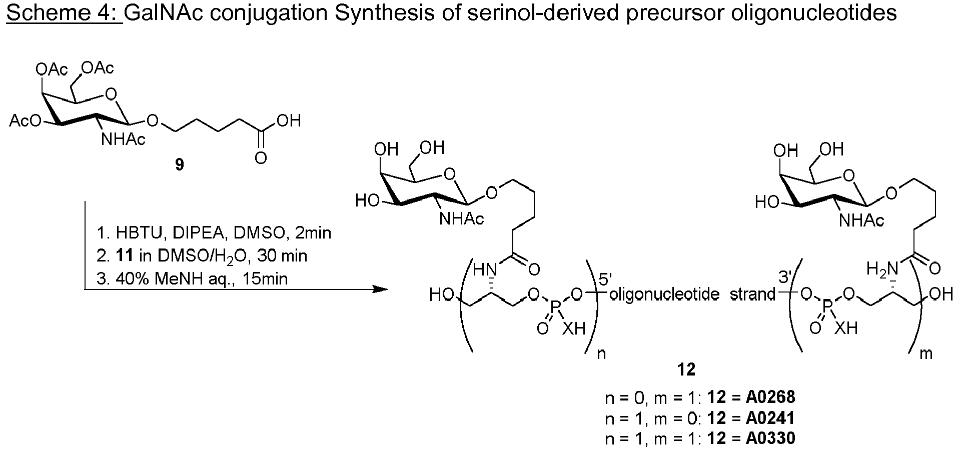

Post solid phase synthesis GalNAc-conjugation was achieved by pre-activation of the GalN(Ac4)-C4-acid (**9**) by a peptide coupling reagent such as HBTU. The pre-activated acid **9** was then reacted with the amino-groups in **11** (e.g. **A0264**) to form the intermediate GalN(Ac4)-conjugates. The acetyl groups protecting the hydroxyl groups in the GalNAc-moieties were cleaved off by methylamine treatment to yield the desired example compounds **12** (e.g. **A0268**), which were further purified by AEX-HPLC and SEC.

### Synthesis of the single stranded tri-antennary GalNAc conjugates in reference conjugates 3-4

Oligonucleotides synthesis of tri-antennary GalNAc-cluster conjugated siRNA is outlined in Figure 9. Oligonucleotide chain assembly is commenced using base loaded support e.g. 5'DMT-2'FdA(bz)-succinate-lcaa-CPG as in example compound **A0006.** Phosphoramidite synthesis coupling cycle consisting of 1) DMT-removal, 2) chain elongation using the required DMT-masked phosphoramidite, 3) capping of non-elongated oligonucleotide chains, followed by oxidation of the P(III) to P(V) either by Iodine or EDITH (if phosphorothioate linkage is desired) and again capping (Cap/Ox/Cap or Cap/Thio/Cap) is repeated until full length of the product is reached. For the on column conjugation of a trivalent tri-antennary GalNAc cluster the same synthesis cycle was applied with using the necessary trivalent branching amidite **C4XLT-phos** followed by another round of the synthesis cycle using the GalNAc amidite **ST23-phos.** Upon completion of this last synthesizer step, the oligonucleotide was cleaved from the solid support and additional protecting groups may be removed by methylamine treatment. The crude products were then purified each by AEX-HPLC and SEC.

### General procedure of double strand formation

In order to obtain the double stranded conjugates, individual single strands are dissolved in a concentration of 60 OD/mL in H₂O. Both individual oligonucleotide solutions can be added together to a reaction vessel. For reaction monitoring a titration can be performed. The first strand is added in 25% excess over the second strand as determined by UV-absorption at 260nm. The reaction mixture is heated e.g. to 80°C for 5min and then slowly cooled to RT. Double strand formation may be monitored by ion pairing reverse phase HPLC. From the UV-area of the residual single strand the needed amount of the second strand can be calculated and added to the reaction mixture. The reaction is heated e.g. to 80°C again and slowly cooled to RT. This procedure can be repeated until less than 10% of residual single strand is detected.

The above process (including Schemes 1-4 and Figures 8 and 9) may be easily adapted to replace GalNac with another targeting ligand e.g. a saccharide.

In any of the above aspects, instead of post solid phase synthesis conjugation it is possible to make a preformed Serinol(GN)-phosphoramidite and use this for on-column conjugation.

### Nucleic Acid

In all cases described herein, the nucleic acid is RNA which forms a double stranded RNA.

The nucleic acid may be a functional nucleic acid, and in particular, the nucleic acid is siRNA.

The siRNA is able to interfere with or inhibit gene expression. Inhibition may be complete or partial and results in down regulation of gene expression in a targeted manner. The nucleic acid comprises two separate polynucleotide strands; the first strand, which may also be a guide strand; and a second strand, which may also be a passenger strand. The first strand may also be referred to as an antisense strand. The second strand may also be referred to as a sense strand.

The nucleic acid comprises a double stranded nucleic acid portion or duplex region formed by all or a portion of the first strand (also known in the art as a guide strand) and all or a portion of the second strand (also known in the art as a passenger strand). The duplex region is defined as beginning with the first base pair formed between the first strand and the second strand and ending with the last base pair formed between the first strand and the second strand, inclusive.

By duplex region it is meant the region in two complementary or substantially complementary oligonucleotides that form base pairs with one another, either by Watson-Crick base pairing or any other manner that allows for a duplex between oligonucleotide strands that are complementary or substantially complementary. The duplex region of a double stranded RNA may range from 15-30 nucleotide base pairs using the Watson-crick base pairing. The duplex region may have 1, 2, 3, 4, 5, 6, 7, 8, 9, 10, 11, 12, 13, 14, 15, 16, 17, 18, 19, 20, 21, 22, 23, 24, 25, 26, 27, 28, 29, 30 base pairs.

Thus, in an embodiment, the nucleic acid portion comprises or consists of two RNA strands of 15-30 based-paired ribonucleotides, suitably 19-25 or 20-25 e.g. 19-23 based-paired ribonucleotides.

Preferably, the nucleic acid has 19-25 e.g. 19 to 23 base pairs. For example, the nucleic acid may be 19, 20, 21, 22 or 23 base pairs in length.

For example, an oligonucleotide strand having 21 nucleotide units can base pair with another oligonucleotide of 21 nucleotide units, yet only 19 nucleotides on each strand are complementary or substantially complementary, such that the "duplex region" consists of 19 base pairs. The remaining base pairs may exist as 5' and 3' overhangs, or as single stranded regions. Overhangs are discussed in more detail below. Further, within the duplex region, 100% complementarity is not required; substantial complementarity is allowable within a duplex region. Substantial complementarity refers to complementarity between the strands such that they are capable of annealing under biological conditions. Techniques to empirically determine if two strands are capable of annealing under biological conditions are well known in the art. Alternatively, two strands can be synthesised and added together under biological conditions to determine if they anneal to one another.

The portion of the first strand and second strand that form at least one duplex region may be fully complementary and are at least partially complementary to each other. Depending on the length of a nucleic acid, a perfect match in terms of base complementarity between the first strand and second strand is not necessarily required. However, the first and second strands must be able to hybridise under physiological conditions. The complementarity may be at least 70%, 75%, 80%, 85%, 90% or 95%.

The first strand and the second strand may each comprise a region of complementarity which comprises at least 15 contiguous nucleotides differing by no more than 3 nucleotides.

The first strand and the target may be fully complementary and are at least partially complementary to each other, preferably fully complementary. The complementarity may be at least 70%, 75%, 80%, 85%, 90%, 95% or 100%, preferably 90%, 95% or 100% (e.g. 100%). The first strand and the target may each comprise a region of complementarity which comprises at least 15 contiguous nucleotides differing by no more than 3 nucleotides.

The nucleic acid of the present invention can be produced using routine methods in the art including chemically synthesis or expressing the nucleic acid either in vitro (e.g., run off transcription) or in vivo. For example, using solid phase chemical synthesis or using an expression vector. In one embodiment, the expression vector can produce the nucleic acid of the invention in a target cell. Methods for the synthesis of the nucleic acid molecule described herein are known to persons skilled in the art.

The ribonucleic acid constructs may be incorporated into suitable vector systems. Preferably the vector comprises a promoter for the expression of RNA. The promoter may be selected from any known in the art such as pol III, U6, H1 or 7SK.

### RNA modifications

Modifications of the RNA molecules of the present invention generally provide a powerful tool in overcoming potential limitations including, but not limited to, in vitro and in vivo stability and bioavailability inherent to native RNA molecules. Modification can further enhance the functional delivery of an siRNA to a target cell. Modified siRNA can also minimize the possibility of activating interferon activity in humans.

The term "modification" indicates a difference from a naturally occurring molecule. The term "modification" does not refer to the conjugation of the present invention, but instead refers to additional modifications which may or may not exist and are described below.

The nucleic acid may be a modified nucleic acid. The modification may be selected from substitutions or insertions with analogues of nucleic acids or bases and chemical modification of the base, sugar or phosphate moieties compared to essentially that which occurs in nature. The modifications that occur in a nucleic acid will be repeated within a polynucleotide molecule such as a modification of a base, or a phosphate moiety, or the non-linking O of a phosphate moiety. A modification may only occur at a 3' or 5' terminal position, may only occur in terminal regions, such as at a position on a terminal nucleotide or in the last 2, 3, 4, 5, or 10 nucleotides of a strand. A modification may occur in a double strand region, a single strand region, or in both. A modification may occur only in the double strand region of a nucleic acid of the invention or may only occur in a single strand region of an nucleic acid of the invention. A phosphorothioate modification at a non-linking O position may only occur at one or both termini, may only occur in a terminal region, e.g., at a position on a terminal nucleotide or in the last 2, 3, 4 or 5 nucleotides of a strand, or may occur in duplex and/or in single strand regions, particularly at termini.

Details of possible modifications according to the invention are described below.

### Modification of nucleotides

Unmodified polynucleotides, particularly ribonucleotides, may be prone to degradation by cellular nucleases, and, as such, modified nucleotides may be included in the nucleic acid of the invention.

Thus, one or more nucleotides of a nucleic acid of the present invention may be modified. One or more nucleotides on the first and/or second strand may be modified, to form modified nucleotides. One or more of the odd numbered nucleotides of the first strand may be modified. One or more of the even numbered nucleotides of the first strand may be modified. One or more of the even numbered nucleotides of the first strand may be modified by at least a second modification, wherein the at least second modification is different from the modification on the one or more odd nucleotides. At least one of the one or more modified even numbered nucleotides may be adjacent to at least one of the one or more modified odd numbered nucleotides.

A plurality of odd numbered nucleotides in the first strand may be modified in the nucleic acid of the invention. A plurality of even numbered nucleotides in the first strand may be modified in the nucleic acid of the invention. A plurality of even numbered nucleotides in the first strand may be modified by a second modification. The first strand may comprise adjacent nucleotides that are modified by a common modification. The first strand may also comprise adjacent nucleotides that are modified by a second different modification.

One or more of the odd numbered nucleotides of the second strand may be modified by a modification that is different to the modification of the odd numbered nucleotides on the first strand and/or one or more of the even numbered nucleotides of the second strand. One or more of the even numbered nucleotides of the second strand may be modified by a modification that is different to the modification of the odd numbered nucleotides on the first strand and/or one or more of the even numbered nucleotides of the second strand. At least one of the one or more modified even numbered nucleotides of the second strand may be adjacent to the one or more modified odd numbered nucleotides. A plurality of odd numbered nucleotides of the second strand may be modified by a common modification and/or a plurality of even numbered nucleotides may be modified by the same modification that is present on the first strand odd numbered nucleotides. A plurality of odd numbered nucleotides on the second strand may be modified by a second modification, wherein the second modification is different from the modification of the first strand odd numbered nucleotides.

One or more or each of the odd numbered nucleotides may be modified in the first strand and one or more or each of the even numbered nucleotides may be modified in the second strand. One or more or each of the even numbered nucleotides may be modified in the first strand and one or more or each of the even numbered nucleotides may be modified in the second strand. One or more or each of the odd numbered nucleotides may be modified in the first strand and one or more of the odd numbered nucleotides may be modified in the second strand by a common modification. One or more or each of the even numbered nucleotides may be modified in the first strand and one or more or each of the odd numbered nucleotides may be modified in the second strand by a common modification. In any of the above embodiments, one or more or each of the alternating nucleotides on either or both strands may be modified by a second modification.

The second strand comprises adjacent nucleotides that are modified by a common modification, which may be a second modification that is different from the modification of the odd numbered nucleotides of the first strand.

In the nucleic acid of the invention, each of the odd numbered nucleotides in the first strand and each of the even numbered nucleotides in the second strand may be modified with a common modification and, each of the even numbered nucleotides may be modified in the first strand with a second modification and each of the odd numbered nucleotides may be modified in the second strand with a second different modification.

The nucleic acid of the invention may have the modified nucleotides of the first strand shifted by at least one nucleotide relative to the unmodified or differently modified nucleotides of the second strand. Modifications to the nucleotides are discussed under *"Modifications to sugar moiety"* below.

The modified nucleotide may be in the duplex region. The modified nucleotide may be outside the duplex region, i.e., in a single stranded region. The modified nucleotide may be on the first strand and may be outside the duplex region. The modified nucleotide may be on the second strand and may be outside the duplex region. The 3'-terminal nucleotide of the first strand may be a modified nucleotide. The 3'-terminal nucleotide of the second strand may be a modified nucleotide. The 5'-terminal nucleotide of the first strand may be a modified nucleotide. The 5'-terminal nucleotide of the second strand may be a modified nucleotide.

An nucleic acid of the invention may have 1 modified nucleotide or a nucleic acid of the invention may have about 2-4 modified nucleotides, or a nucleic acid may have about 4-6 modified nucleotides, about 6-8 modified nucleotides, about 8-10 modified nucleotides, about 10-12 modified nucleotides, about 12-14 modified nucleotides, about 14-16 modified nucleotides about 16-18 modified nucleotides, about 18-20 modified nucleotides, about 20-22 modified nucleotides, about 22-24 modified nucleotides, 24-26 modified nucleotides or about 26-28 modified nucleotides. In each case the nucleic acid comprising said modified nucleotides retains at least 50% of its activity as compared to the same nucleic acid but without said modified nucleotides. The nucleic acid may retain 55%, 60%, 65%, 70%, 75%, 80%, 85%, 90%, 95% or 100% or above of its activity as compared to the same nucleic acid but without said modified nucleotides

The modified nucleotide may comprise a purine or a pyrimidine base. At least half of the purines may be modified. At least half of the pyrimidines may be modified. All of the purines may be modified. All of the pyrimidines may be modified.

The nucleic acid may comprise a nucleotide comprising a modified nucleotide selected from 2-aminoadenosine, 2,6-diaminopurine riboside, inosine, pyridin-4-one riboside, pyridin-2-one riboside, phenyl riboside, pseudouridine, 2,4,6-trimethoxy benzene riboside, 3-methyl uridine, dihydrouridine, naphthyl, aminophenyl riboside, 5-alkylcytidine (e.g., 5-methylcytidine), 5-alkyluridine (e.g., ribothymidine), 5-halouridine (e.g., 5-bromouridine), 6-azapyrimidine riboside, 6-alkylpyrimidine riboside (e.g. 6-methyluridine), propyne riboside (e.g. 5-(1-propynyl)-2'-deoxy-Uridine (pdU) or 5-(1-propynyl)-2'-deoxyCytidine (pdC)), queuosine, 2-thiouridine, 4-thiouridine, wybutosine, wybutoxosine, 4-acetylcytidine, 5-(carboxyhydroxymethyl)uridine, 5'-carboxymethylaminomethyl-2-thiouridine, 5-carboxymethylaminomethyluridine, beta-D-galactosylqueuosine, 1-methyladenosine, 1-methylinosine, 2,2-dimethylguanosine, 3-methylcytidine, 2-methyladenosine, 2-methylguanosine, N6-methyladenosine, 7-methylguanosine, 5-methoxyaminomethyl-2-thiouridine, 5-methylaminomethyluridine, 5-methylcarbonylmethyluridine, 5-methyloxyuridine, 5-methyl-2-thiouridine, 2-methylthio-N6-isopentenyladenosine, beta-D-mannosylqueuosine, uridine-5-oxyacetic acid and 2-thiocytidine.

The nucleic acids of the invention may be included one or more inverted nucleotides, for example inverted thymidine or inverted adenine (for example see Takei, et al., 2002. JBC 277 (26):23800-06). In particular, the nucleic acids of the invention may comprise a modification wherein the terminal nucleotide at the 3' end of at least one of the first strand and the second strand is an inverted nucleotide and is attached to the adjacent nucleotide via the 3' carbon of the terminal nucleotide and the 3' carbon of the adjacent nucleotide and/ or the terminal nucleotide at the 5' end of at least one of the first strand and the second strand is an inverted nucleotide and is attached to the adjacent nucleotide via the 5' carbon of the terminal nucleotide and the 5' carbon of the adjacent nucleotide. For example, the inverted nucleotide at the 3' end of at least one of the first strand and the second strand and/ or the inverted nucleotide at the 5' end of at least one of the first strand and the second strand is a purine, such as an adenine.

### Modification of the 3' and/or 5' ends

As explained above, the ends of the RNA strands may be conjugated. Additionally or alternatively to the conjugation described above, the ends of the RNA strands may be modified as described below.

The RNA of the present invention may include nucleic acid molecules comprising one or more modified nucleotides, abasic nucleotides, acyclic or deoxyribonucleotide (2' deoxy) at the terminal 5'- or 3'-end on either or both of the sense or antisense strands.

When the modification is a 2' deoxy modification, only a small portion of the nucleotides may have this modification, for example less than 15%, less than 10% or less than 5%.

Terminal modifications can be added for a number of reasons, including to modulate activity or to modulate resistance to degradation.

In one embodiment, the 5'- and 3'-end nucleotides of both the sense and antisense strands are unmodified. In another embodiment, the 5'-end nucleotide of the antisense strand is modified as described above. In another embodiment, the 5'-end nucleotide of the sense strand is modified as described above. In another embodiment, the 3'-end nucleotide of the antisense strand is modified as described above. In another embodiment, the 3'-end nucleotide of the sense strand is modified as described above. In another embodiment, the 5'-end nucleotide of the antisense strand and the 5'-end nucleotide of the sense strand are modified as described above. In another embodiment, the 3'-end nucleotide of the antisense strand and the 3'-end nucleotide of the sense strand are modified as described above. In another embodiment, the 5'-end nucleotide of the antisense strand and the 3'-end nucleotide of the sense strand are modified as described above. In another embodiment, the 3'-end nucleotide of the antisense strand and the 5'-end nucleotide of the sense strand are modified as described above. In another embodiment, the 3'-end nucleotide of the sense strand and the 5'-end nucleotide of the sense strand are modified as described above. In another embodiment, the 3'-end nucleotide of the antisense strand and both the 5'- and 3'-end nucleotides of the sense strand are modified. Both the 5'- and 3'-end nucleotides of the antisense strand may be modified as described above. In another embodiment, both the 5'- and 3'-end nucleotides of the sense strand are modified as described above.

Examples of different kinds of end modification(s) are presented in **Table 1.**

**Table 1 - Examples of end modifications**

| | | |
|---|---|---|
| | **Antisense strand** | **Sense strand** |
| **1. 5'-end** | free OH | free OH |
| **3'-end** | free OH | free OH |
| | | |
| **2. 5'-end** | free OH | free OH |
| **3'-end** | end modification | end modification |
| | | |
| **3. 5'-end** | free OH | free OH |
| **3'-end** | free OH | end modification |
| | | |
| **4. 5'-end** | free OH | free OH |
| **3'-end** | end modification | free OH |
| | | |
| **5. 5'-end** | free OH | end modification |
| **3'-end** | free OH | free OH |
| | | |
| **6. 5'-end** | free OH | end modification |
| **3'-end** | end modification | free OH |
| | | |
| **7.5'-end** | free OH | end modification |
| **3'-end** | free OH | end modification |
| | | |
| **8. 5'-end** | free OH | end modification |
| **3'-end** | end modification | end modification |
| | | |
| **9. 5'end** | end modification | free OH |
| **3' end** | free OH | free OH |
| | | |
| **10. 5' end** | end modification | end modification |
| **3' end** | free OH | free OH |
| | | |
| **11. 5' end** | end modification | free OH |
| **3' end** | free OH | end modification |

### 1. Blunt ends and overhangs

The double stranded RNAs may be blunt ended at one end or on both ends e.g. blunt on both ends. Alternatively, the double stranded RNAs have an overhang at one end and a blunt end at the other. Alternatively, the double stranded RNAs have an overhang at both ends.

An "overhang" as used herein has its normal and customary meaning in the art, i.e. a single stranded portion of a nucleic acid that extends beyond the terminal nucleotide of a complementary strand in a double strand nucleic acid. Stability of a nucleic acid of the invention may be increased by including particular bases in overhangs, or to include modified nucleotides, in single strand overhangs, e.g., in a 5' or 3' overhang, or in both. Purine nucleotides may be included in overhangs. All or some of the bases in a 3' or 5' overhang may be modified. Modifications can include the use of modifications at the 2' OH group of the ribose sugar and modifications in the phosphate group, such as phosphorothioate modifications. Overhangs need not be homologous with the target sequence.

The term "blunt end" includes double stranded nucleic acid whereby both strands terminate at the same position, regardless of whether the terminal nucleotide(s) are base paired. The terminal nucleotide of a first strand and a second strand at a blunt end may be base paired. The terminal nucleotide of a first strand and a second strand at a blunt end may not be paired. The terminal two nucleotides of a first strand and a second strand at a blunt end may be base paired. The terminal two nucleotides of a first strand and a second strand at a blunt end may not be paired.

When the double stranded RNAs have an overhang, the double stranded RNAs may have overhangs of 1 or more nucleotides one or both strands at one or both ends. The overhangs may be 1, 2, 3, 4, 5, 6, 7, 8, 9, or 10 nucleotides in length.

The overhang may comprise at least one deoxyribonucleotides and/or TT dinucleotide.

The nucleic acid may be blunt ended at the end with the 5' end of the first strand and the 3' end of the second strand or at the 3'-end of the first strand and the 5' end of the second strand.

The nucleic acid may comprise an overhang at a 3' or 5' end. The nucleic acid may have a 3' overhang on the first strand. The nucleic acid may have a 3' overhang on the second strand.

The nucleic acid may have a 5' overhang on the first strand. The nucleic acid may have a 5' overhang on the second strand. The nucleic acid may have an overhang at both the 5' end and 3' end of the first strand. The nucleic acid may have an overhang at both the 5' end and 3' end of the second strand. The nucleic acid may have a 5' overhang on the first strand and a 3' overhang on the second strand. The nucleic acid may have a 3' overhang on the first strand and a 5' overhang on the second strand. The nucleic acid may have a 3' overhang on the first strand and a 3' overhang on the second strand. The nucleic acid may have a 5' overhang on the first strand and a 5' overhang on the second strand.

An overhang at the 3'-end or 5' end of the second strand or the first strand may be selected from consisting of 1, 2, 3, 4 and 5 nucleotides in length. Optionally, an overhang may consist of 1 or 2 nucleotides, which may or may not be modified.

### 2. Phosphorylation

The 5'-end nucleotide of the antisense strand may be phosphorylated. In another embodiment, the 5'-end nucleotide of the sense strand may be phosphorylated. In another embodiment, the 5'-end nucleotides of both the antisense strand and the sense strand are phosphorylated. In another embodiment, the 5'-end nucleotide of the antisense strand is phosphorylated and the 5'-end nucleotide of the sense strand has a free hydroxyl group (5'-OH). In another embodiment, the 5'-end nucleotide of the antisense strand is phosphorylated and the 5'-end nucleotide of the sense strand is modified. In another embodiment the 5'-end nucleotide of the antisense strand carries a 5'-(E)-vinylphosphonate.

Nucleic acids of the invention, on the first or second strand, may be 5' phosphorylated or include a phosphoryl analog at the 5' prime terminus. 5'-phosphate modifications include those which are compatible with RISC mediated gene silencing. Suitable modifications include: 5'-monophosphate ((HO)2(O)P-O-5'); 5'-diphosphate ((HO)2(O)P-O-P(HO)(O)-O-5'); 5'-triphosphate ((HO)2(O)P-O-(HO)(O)P-O-P(HO)(O)-O-5'); 5'-guanosine cap (7-methylated or non-methylated) (7m-G-O-5'-(HO)(O)P-O-(HO)(O)P-O-P(HO)(O)-O-5'); 5'-adenosine cap (Appp), and any modified or unmodified nucleotide cap structure (N-O-5'-(HO)(O)P-O-(HO)(O)P-O-P(HO)(O)-O-5'); 5'-monothiophosphate (phosphorothioate; (HO)2(S)P-O-5'); 5'-monodithiophosphate (phosphorodithioate; (HO)(HS)(S)P-O-5'), 5'-phosphorothiolate ((HO)2(O)P-S-5'); any additional combination of oxygen/sulfur replaced monophosphate, diphosphate and triphosphates (e.g., 5'-alpha-thiotriphosphate, 5'-gamma-thiotriphosphate, etc.), 5'-phosphoramidates ((HO)2(O)P-NH-5', (HO)(NH2)(O)P-O-5'), 5'-alkylphosphonates (R=alkyl=methyl, ethyl, isopropyl, propyl, etc., e.g., RP(OH)(O)-O-5'-, (OH)2(O)P-5'-CH2-), 5'vinylphosphonate, 5'-alkyletherphosphonates (R=alkylether=methoxymethyl (MeOCH2-), ethoxymethyl, etc., e.g., RP(OH)(O)-O-5'-).

### 3. Capping with small chemical groups

The nucleic acids according to the present invention may be modified by capping of the 3' and/or 5' ends of either strand with small chemical groups. Examples of modifications to end nucleotides include, but are not limited to, biotin, abasics, amino, fluoro, chloro, bromo, CN, CF, methoxy, imidazole, carboxylate, thioate, C₁ to C₁₀ alkyl (e.g. C₁ to C₆ alkyl, e.g. methyl, ethyl, propyl), substituted lower alkyl, alkaryl or arylalkyl, OCF₃, OCN, O-, S-, or N-alkyl; O-, S-, or N-alkenyl; SO-CH₃; SO₂CH₃; ONO₂; NO₂, N₃; heterocycloalkyl; heterocycloalkaryl; aminoalkylamino; polyalkylamino or substituted silyl, as, among others, described, e.g., in WO 99/54459, EP 0 586 520 B1 or EP 0 618 925 B1, incorporated by reference in their entireties.

In another aspect, the 5'-end of the antisense strand, the 5'- end of the sense strand, the 3'-end of the antisense strand or the 3'-end of the sense strand may be covalently connected to a prodrug moiety. In one embodiment, the moiety may be cleaved in an endosome. In another the moiety may be cleaved in the cytoplasm.

### 4. Phosphorothioate internucleotide linkages

The nucleic acids according to the present invention may comprise one or more phosphorothioate internucleotide linkage on one or more of the terminal ends of the first and/or the second strand. By phosphorothioate internucleotide linkages it is meant that the linkage between the nucleotide and the adjacent nucleotide comprises a phosphorothioate group instead of a standard phosphate group. Preferably the phosphorothioate internucleotide linkages may be distributed across the entire nucleotide sequences and may occur in any number at any position. Preferably the nucleic acids can comprise between one to ten phosphorothioate internucleotide linkages. Preferably the first RNA strand (i.e. the antisense strand) has one to six phosphorothioate internucleotide linkages at each end. Preferably the second RNA strand (i.e. the sense strand) has one to six phosphorothioate internucleotide linkages at each end. Preferably the antisense strand has at least 1 phosphorothioate modification at each end. Preferably the antisense strand has 1-3 phosphorothioate modifications at each end. Most preferably the antisense strand has 2 phosphorothioate modifications at each end. Preferably the sense strand has at least 1 phosphorothioate modification at the 3' end. Preferably the sense strand has 1-3 phosphorothioate modifications at the 3' end. Most preferably the sense strand has 2 phosphorothioate modifications at the 3' end.

### 5. Phosphorodithioate internucleotide linkages

The nucleic acids according to the present invention may comprise one or more phosphorodithioate internucleotide linkage on one or more of the terminal ends of the first and/or the second strand. By phosphorodithioate internucleotide linkages it is meant that the linkage between the nucleotide and the adjacent nucleotide comprises a phosphorodithioate group instead of a standard phosphate group. Preferably the phosphorodithioate internucleotide linkages may be distributed across the entire nucleotide sequences and may occur in any number at any position. Preferably the nucleic acids can comprise between one to ten phosphorodithioate internucleotide linkages. Preferably the first RNA strand (i.e. the antisense strand) has one to six phosphorodithioate internucleotide linkages at each end. Preferably the second RNA strand (i.e. the sense strand) has one to six phosphorodithioate internucleotide linkages at each end. Preferably the antisense strand has at least 1 phosphorodithioate modification at the 3' end. Preferably the antisense strand has 1-3 phosphorodithioate modifications at the 3' end. Most preferably the antisense strand has 1 phosphorodithioate modifications at the 3'end. Preferably the sense strand has 1-3 phosphorodithioate modification at both ends. Preferably the sense strand has at least 1 phosphorodithioate modification at both ends. Preferably the sense strand has at least 1 phosphorodithioate modification at the 3' end. Preferably the sense strand has 1-3 phosphorodithioate modifications at the 3' end. Most preferably the sense strand has 1 phosphorodithioate modification at the 3' end.

### Labelling and Protecting groups

The 3' and 5' ends of an oligonucleotide can be conjugated to other functional molecular entities such as labelling moieties, e.g., fluorophores (e.g., pyrene, TAMRA, fluorescein, an Alexa dye, Cy3 or Cy5 dyes) or protecting groups (based e.g., on sulfur, silicon, boron or ester).

The functional molecular entities can be attached to the sugar through a phosphate group and/or a linker. The terminal atom of the linker can connect to or replace the linking atom of the phosphate group or the C-3' or C-5' O, N, S or C group of the sugar. Alternatively, the linker can connect to or replace the terminal atom of a nucleotide surrogate (e.g., PNAs). These spacers or linkers can include e.g., -(CH2)n-, -(CH2)nN-, -(CH2)nO-,-(CH2)nS-, O(CH2CH2O)nCH2CH2OH (e.g., n=3 or 6), abasic sugars, amide, carboxy, amine, oxyamine, oxyimine, thioether, disulfide, thiourea, sulfonamide, or morpholino, or biotin and fluorescein reagents. The 3' end can be an -OH group.

### 6. Miscellaneous end modifications

Terminal modifications can also be useful for enhancing uptake. Useful modifications for this include modifying the terminal ends of either strand with cholesterol. Terminal modifications can also be useful for cross-linking an RNA agent to another moiety.

Other examples of terminal modifications include dyes, intercalating agents (e.g., acridines), cross-linkers (e.g., psoralene, mitomycin C), porphyrins (TPPC4, texaphyrin, Sapphyrin), polycyclic aromatic hydrocarbons (e.g., phenazine, dihydrophenazine), artificial endonucleases (e.g., EDTA), lipophilic carriers (e.g., cholesterol, cholic acid, adamantane acetic acid, 1-pyrene butyric acid, dihydrotestosterone, 1,3-Bis-O(hexadecyl)glycerol, geranyloxyhexyl group, hexadecylglycerol, borneol, menthol, 1,3-propanediol, heptadecyl group, palmitic acid, myristic acid, O3-(oleoyl)lithocholic acid, O3-(oleoyl)cholenic acid, dimethoxytrityl, or phenoxazine) and peptide conjugates (e.g., antennapedia peptide, Tat peptide), alkylating agents, phosphate, amino, mercapto, PEG (e.g., PEG-40K), MPEG, [MPEG]2, polyamino, alkyl, substituted alkyl, radiolabeled markers, enzymes, haptens (e.g., biotin), transport/absorption facilitators (e.g., aspirin, vitamin E, folic acid), synthetic ribonucleases (e.g., imidazole, bisimidazole, histamine, imidazole clusters, acridine-imidazole conjugates, Eu3+ complexes of tetraazamacrocycles).

Certain moieties may be linked to the 5' terminus of the first strand or the second strand and includes abasic ribose moiety, abasic deoxyribose moiety, modifications abasic ribose and abasic deoxyribose moieties including 2' O alkyl modifications; inverted abasic ribose and abasic deoxyribose moieties and modifications thereof, C6-imino-Pi; a mirror nucleotide including L-DNA and L-RNA; 5'OMe nucleotide; and nucleotide analogs including 4',5'-methylene nucleotide; 1-(beta-D-erythrofuranosyl)nucleotide; 4'-thio nucleotide, carbocyclic nucleotide; 5'-amino-alkyl phosphate; 1,3-diamino-2-propyl phosphate, 3-aminopropyl phosphate; 6-aminohexyl phosphate; 12-aminododecyl phosphate; hydroxypropyl phosphate; 1,5-anhydrohexitol nucleotide; alpha-nucleotide; threo-pentofuranosyl nucleotide; acyclic 3',4'-seco nucleotide; 3,4-dihydroxybutyl nucleotide; 3,5-dihydroxypentyl nucleotide, 5'-5'-inverted abasic moiety; 1,4-butanediol phosphate; 5'-amino; and bridging or non bridging methylphosphonate and 5'-mercapto moieties.

### Modifications to the base

One or more nucleotides of a RNA of the present invention may comprise a modified base. Adenine, guanine, cytosine and uracil are the most common bases found in RNA. These bases can be modified or replaced to provide RNAs having improved properties. E.g., nuclease resistant oligoribonucleotides can be prepared with these bases or with synthetic and natural nucleobases (e.g., inosine, thymine, xanthine, hypoxanthine, nubularine, isoguanisine, or tubercidine) and any one of the above modifications. Alternatively, substituted or modified analogs of any of the above bases and "universal bases" can be employed.

In one aspect, the RNA comprises at least one nucleotide comprising a modified base. In one embodiment, the modified base is on the antisense strand. In another embodiment, the modified base is on the sense strand. In another embodiment, the modified base is in the duplex region. In another embodiment, the modified base is outside the duplex region, i.e., in a single stranded region. In another embodiment, the modified base is on the antisense strand and is outside the duplex region. In another embodiment, the modified base is on the sense strand and is outside the duplex region. In another embodiment, the 3'-terminal nucleotide of the antisense strand is a nucleotide with a modified base. In another embodiment, the 3'-terminal nucleotide of the sense strand is nucleotide with a modified base. In another embodiment, the 5'-terminal nucleotide of the antisense strand is nucleotide with a modified base. In another embodiment, the 5'-terminal nucleotide of the sense strand is nucleotide with a modified base.

In one embodiment, a RNA may have 1 modified base. In another embodiment, a RNA may have about 2-4 modified bases. In another embodiment, a RNA has about 4-6 modified bases. In another embodiment, a RNA has about 6-8 modified bases. In another embodiment, a RNA has about 8-10 modified bases. In another embodiment, a RNA has about 10-12 modified bases. In another embodiment, a RNA has about 12-14 modified bases. In another embodiment, a RNA has about 14-16 modified bases. In another embodiment, a RNA has about 16-18 modified bases. In another embodiment, a RNA has about 18-20 modified bases. In another embodiment, a RNA has about 20-22 modified bases. In another embodiment, a RNA has about 22-24 modified bases. In another embodiment, a RNA has about 24-26 modified bases. In another embodiment, a RNA has about 26-28 modified bases. In each case the RNA comprising said modified bases retains at least 50% of its activity as compared to the same RNA but without said modified bases.

Examples of modified bases include 2-aminoadenine, 6-methyl and other alkyl derivatives of adenine and guanine, 2-propyl and other alkyl derivatives of adenine and guanine, 5-halouracil and cytosine, 5-propynyl uracil and cytosine, 6-azo uracil, cytosine and thymine, 5-uracil (pseudouracil), 4-thiouracil, 5-halouracil, 5-(2-aminopropyl)uracil, 5-amino allyl uracil, 8-halo, amino, thiol, thioalkyl, hydroxyl and other 8-substituted adenines and guanines, 5-trifluoromethyl and other 5-substituted uracils and cytosines, 7-methylguanine, 5-substituted pyrimidines, 6-azapyrimidines and N-2, N-6 and O-6 substituted purines, including 2-aminopropyladenine, 5-propynyluracil and 5-propynylcytosine, dihydrouracil, 3-deaza-5-azacytosine, 2-aminopurine, 5-alkyluracil, 7-alkylguanine, 5-alkyl cytosine, 7-deazaadenine, N6,N6-dimethyladenine, 2,6-diaminopurine, 5-amino-allyl-uracil, N3-methyluracil, substituted 1,2,4-triazoles, 2-pyridinone, 5-nitroindole, 3-nitropyrrole, 5-methoxyuracil, uracil-5-oxyacetic acid, 5-methoxycarbonylmethyluracil, 5-methyl-2-thiouracil, 5-methoxycarbonylmethyl-2-thiouracil, 5-methylaminomethyl-2-thiouracil, 3-(3-amino-3-carboxypropyl)uracil, 3-methylcytosine, 5-methylcytosine, N4-acetyl cytosine, 2-thiocytosine, N6-methyladenine, N6-isopentyladenine, 2-methylthio-N6-isopentenyladenine, N-methylguanines, or O-alkylated bases.

The modified base may be a modified purine or a pyrimidine. In another embodiment, at least half of the purines are modified. In another embodiment, at least half of the pyrimidines are modified. In another embodiment, all of the purines are modified. In another embodiment, all of the pyrimidines are modified. In another embodiment, the RNA may comprise a nucleotide comprising a modified base as described above.

In another aspect, a RNA of the present invention comprises an abasic nucleotide. The term "abasic" as used herein, refers to moieties lacking a base or having other chemical groups in place of a base at the 1' position, for example a 3',3'-linked or 5',5'-linked deoxyabasic ribose derivative. As used herein, a nucleotide with a modified base does not include abasic nucleotides. In one aspect, the RNA comprises at least one abasic nucleotide. In one embodiment, the abasic nucleotide is on the antisense strand. In another embodiment, the abasic nucleotide is on the sense strand. In another embodiment, the abasic nucleotide is in the duplex region. In another embodiment, the abasic nucleotide is outside the duplex region. In another embodiment, the abasic nucleotide is on the antisense strand and is outside the duplex region. In another embodiment, the abasic nucleotide is on the sense strand and is outside the duplex region. In another embodiment, the 3'-terminal nucleotide of the antisense strand is an abasic nucleotide. In another embodiment, the 3'-terminal nucleotide of the sense strand is an abasic nucleotide. In another embodiment, the 5'-terminal nucleotide of the antisense strand is an abasic nucleotide. In another embodiment, the 5'-terminal nucleotide of the sense strand is an abasic nucleotide. In another embodiment, a RNA has a number of abasic nucleotides selected from 1, 2, 3, 4, 5 and 6.

### Modifications to sugar moiety

Another aspect relates to modifications of a sugar moiety. One or more nucleotides of a RNA of the present invention may comprise a modified ribose moiety. Modifications at the 2'-position where the 2'-OH is substituted include the non-limiting examples selected from alkyl, substituted alkyl, alkaryl-, arylalkyl-, -F, -CI, -Br, -CN, -CF3, -OCF3, -OCN, -O-alkyl, -S-alkyl, HS-alkyl-O, -O-alkenyl, -S-alkenyl, -N-alkenyl, -SO-alkyl, -alkyl-OSH, -alkyl-OH, -O-alkyl-OH, -O-alkyl-SH, -S-alkyl-OH, -S-alkyl-SH, -alkyl-S-alkyl, -alkyl-O-alkyl, -ONO2, -NO2, -N3, -NH2, alkylamino, dialkylamino-, aminoalkyl-, aminoalkoxy, aminoacid, aminoacyl-, -ONH2, -O-aminoalkyl, -O-aminoacid, -O-aminoacyl, heterocycloalkyl-, heterocycloalkaryl-, aminoalkylamino-, polyalklylamino-, substituted silyl-, methoxyethyl- (MOE), alkenyl and alkynyl. "Locked" nucleic acids (LNA) in which the 2' hydroxyl is connected, e.g., by a methylene bridge, to the 4' carbon of the same ribose sugar is further included as a 2' modification of the present invention. Preferred substituents are 2'-methoxyethyl, 2'-O-CH3, 2'-O-allyl, 2'-C-allyl, and 2'-fluoro.

The modification and / or modifications may each and individually be selected from the group consisting of 3' terminal deoxy thymine, 2' O methyl, a 2' deoxy modification, a 2' amino modification, a 2' alkyl modification, a morpholino modification, a phosphoramidate modification, 5'-phosphorothioate group modification, a 5' phosphate or 5' phosphate mimic modification and a cholesteryl derivative or a dodecanoic acid bisdecylamide group modification and/or the modified nucleotide may be any one of a locked nucleotide, an abasic nucleotide (which lack a nucleobase at C-I') or a non natural base comprising nucleotide. At least one modification may be 2'-O-methyl and/or at least one modification may be 2'-F.

When the modification is a 2' deoxy modification, only a small portion of the nucleotides may have this modification, for example less than 15%, less than 10% or less than 5%.

In one embodiment, the RNA comprises 1-5 2'-modified nucleotides. In another embodiment, the RNA comprises 5-10 2'-modified nucleotides. In another embodiment, the RNA comprises 15-20 2'-modified nucleotides. In another embodiment, the RNA comprises 20-25 2'-modified nucleotides. In another embodiment, the RNA comprises 25-30 2'-modified nucleotides. In an embodiment, the sugar group can also contain one or more carbons that possess the opposite stereochemical configuration than that of the corresponding carbon in ribose. Thus, a modified nucleotides may contain a sugar such as arabinose.

In one embodiment, the RNA comprises 1-5 2'-O-CH3 modified nucleotides. In another embodiment, the RNA comprises 5-10 2'-O-CH3 modified nucleotides. In another embodiment, the RNA comprises 15-20 2'-O-CH3 modified nucleotides. In another embodiment, the RNA comprises 20-25 2'-O-CH3 modified nucleotides. In another embodiment, the RNA comprises 25-30 2'-O-CH3 modified nucleotides.

In one embodiment, the RNA duplex region comprises 1-5 2'-O-CH3 modified nucleotides. In another embodiment, the RNA duplex region comprises 5-10 2'-O-CH3 modified nucleotides. In another embodiment, the RNA duplex region comprises 15-20 2'-O-CH3 modified nucleotides. In another embodiment, the RNA duplex region comprises 20-25 2'-O-CH3 modified nucleotides. In another embodiment, the RNA duplex region comprises 25-30 2'-O-CH3 modified nucleotides.

In one embodiment, the RNA comprises an antisense strand of 19 nucleotides in length and a sense strand 19 nucleotides in length.

In another embodiment, the RNA comprises an antisense strand 20 nucleotides in length and a sense strand 20 nucleotides in length.

In another embodiment, the RNA comprises an antisense strand 21 nucleotides in length and a sense strand 21 nucleotides in length.

In another embodiment, the RNA comprises an antisense strand 22 nucleotides in length and a sense strand 22 nucleotides in length.

In another embodiment, the RNA comprises an antisense strand 23 nucleotides in length and a sense strand 23 nucleotides in length.

In any of the above embodiments, the antisense strand comprises 2'-O-CH3 modifications at nucleotides 1, 3, 5, 7, 9, 11, 13, 15, 17, 19, 21 and 23, and the sense strand comprises 2'-O-CH3 modifications at nucleotides 2, 4, 6, 8, 10, 12 ,14, 16, 18, 20 and 22 wherein said antisense strand is numbered from 5'-3' and said sense strand is numbered from 3'-5'.

In another embodiment, the RNA comprises an antisense strand 18-23 nucleotides in length and a sense strand 18-23 nucleotides in length, wherein said antisense strand comprises 2'-O-CH3 modifications at nucleotides 3, 5, 7, 9, 11, 13, 15 and 17 (such as 5, 7, 9, 11, 13 and 15, e.g. 7, 9, 11, 13), and wherein said sense strand comprises 2'-O-CH3 modifications at nucleotides 4, 6, 8, 10, 12 ,14 and 16 (such as 6, 8, 10, 12 and 14 e.g. 8, 10 and 12), wherein said antisense strand is numbered from 5'-3' and said sense strand is numbered from 3'-5'.

In another embodiment, the RNA comprises an antisense strand 18-23 nucleotides in length and a sense strand 18-23 nucleotides in length, wherein said antisense strand comprises 2'-O-CH3 modifications at nucleotides 7, 9 and 11, and wherein said sense strand comprises 2'-O-CH3 modifications at nucleotides 8, 10 and 12, wherein said antisense strand is numbered from 5'-3' and said sense strand is numbered from 3'-5'.

In another embodiment, the RNA comprises an antisense strand 18-23 nucleotides in length and a sense strand 18-23 nucleotides in length, wherein said antisense strand comprises 2'-O-CH3 modifications at nucleotides 7 and 9, and wherein said sense strand comprises 2'-O-CH3 modifications at nucleotides 8 and 10, wherein said antisense strand is numbered from 5'-3' and said sense strand is numbered from 3'-5'.

In another embodiment, the RNA comprises an antisense strand 18-23 nucleotides in length and a sense strand 18-23 nucleotides in length, wherein said antisense strand comprises 2'-O-CH3 modifications at nucleotides 9 and 11, and wherein said sense strand comprises 2'-O-CH3 modifications at nucleotides 8 and 10, wherein said antisense strand is numbered from 5'-3' and said sense strand is numbered from 3'-5'.

### Modifications to phosphate backbone

Another aspect relates to modifications to a phosphate backbone. All or a portion of the nucleotides of the RNA of the invention may be linked through phosphodiester bonds, as found in unmodified nucleic acid. A RNA of the present invention however, may comprise a modified phosphodiester linkage. The phosphodiester linkages of either the antisense stand or the sense strand may be modified to independently include at least one heteroatom selected from nitrogen and sulfur. In one embodiment, a phosphodiester group connecting a ribonucleotide to an adjacent ribonucleotide is replaced by a modified group.

In one embodiment, all of the nucleotides of the antisense strand are linked through phosphodiester bonds. In another embodiment, all of the nucleotides of the antisense duplex region are linked through phosphodiester bonds. In another embodiment, all of the nucleotides of the sense strand are linked through phosphodiester bonds. In another embodiment, all of the nucleotides of the sense duplex region are linked through phosphodiester bonds. In another embodiment, the antisense strand comprises a number of modified phosphodiester groups selected from 1, 2, 3, 4, 5, 6, 7, 8, 9 or 10. In another embodiment, the antisense duplex region comprises a number of modified phosphodiester groups selected from 1, 2, 3, 4, 5, 6, 7, 8, 9 or 10. In another embodiment, the sense strand comprises a number of modified phosphodiester groups selected from 1, 2, 3, 4, 5, 6, 7, 8, 9 or 10. In another embodiment, the sense duplex region comprises a number of modified phosphodiester groups selected from 1, 2, 3, 4, 5, 6, 7, 8, 9 or 10.

Examples of modified phosphate groups include phosphorothioate, phosphoroselenates, borano phosphates, borano phosphate esters, hydrogen phosphonates, phosphoroamidates, alkyl or aryl phosphonates and phosphotriesters. Phosphorodithioates have both non-linking oxygens replaced by sulphur. One, each or both non-linking oxygens in the phosphate group can be independently any one of S, Se, B, C, H, N, or OR (R is alkyl or aryl).

The phosphate linker can also be modified by replacement of a linking oxygen with nitrogen (bridged phosphoroamidates), sulfur (bridged phosphorothioates) and carbon (bridged methylenephosphonates). The replacement can occur at a terminal oxygen. Replacement of the non-linking oxygens with nitrogen is possible.

The phosphate linker and ribose sugar may be replaced by nuclease resistant nucleotides.

Examples include the mophilino, cyclobutyl, pyrrolidine and peptide nucleic acid (PNA) nucleoside surrogates. In certain embodiments, PNA surrogates may be used.

### Flanking Groups

In one aspect, the antisense duplex region comprises a plurality of groups of modified nucleotides, referred to herein as "modified groups", wherein each modified group consists of one or more identically modified nucleotides, wherein each modified group is flanked on one or both sides by a second group of nucleotides, referred to herein as "flanking groups", wherein each said flanking group consists of one or more nucleotides that are either unmodified or modified in a manner different from the nucleotides of said modified group. In one embodiment, each modified group in the antisense duplex region is identical, i.e., each modified group consists of an equal number of identically modified nucleotides. In another embodiment, each flanking group has an equal number of nucleotides. In another embodiment, each flanking group is identical. In another embodiment, the nucleotides of said modified groups in the antisense duplex region comprise a modified base. In another embodiment, the nucleotides of said modified groups comprise a modified phosphate backbone. In another embodiment, the nucleotides of said modified groups comprise a modified 2' position.

In another aspect, the sense duplex region comprises a plurality of groups of modified groups, wherein each modified group consists of one or more identically modified nucleotides, wherein each modified group is flanked on one or both sides by a flanking group, wherein each said flanking group consists of one or more nucleotides that are either unmodified or modified in a manner different from the nucleotides of said modified group. In one embodiment, each modified group in the sense duplex region is identical. In another embodiment, each flanking group has an equal number of nucleotides. In another embodiment, each flanking group is identical. In another embodiment, the nucleotides of said modified groups in the sense duplex region comprise a modified base. In another embodiment, the nucleotides of said modified groups comprise a modified phosphate backbone. In another embodiment, the nucleotides of said modified groups comprise a modified 2' position.

In another aspect, the antisense duplex region and the sense duplex region each comprise a plurality of modified groups, wherein each modified group consists of one or more identically modified nucleotides, wherein each modified group is flanked on one or both sides by a flanking group, wherein each said flanking group consists of one or more nucleotides that are either unmodified or modified in a manner different from the nucleotides of said modified group. In one embodiment, each modified group in the antisense duplex region and the sense duplex region are identical. In another embodiment, each flanking group in the antisense duplex region and the sense duplex region each have an equal number of nucleotides. In another embodiment, each flanking group in the antisense duplex region and in the sense duplex region are identical. In another embodiment, the nucleotides of said modified groups in the antisense duplex region and the sense duplex region each comprise the same modified groups and the same flanking groups. In another embodiment, the nucleotides of said modified groups in the antisense duplex region and the sense duplex region each comprise a modified base. In another embodiment, the nucleotides of said modified groups in the antisense duplex region and the sense duplex region each comprise a modified phosphate backbone. In another embodiment, the nucleotides of said modified groups in the antisense duplex region and the sense duplex region each comprise a modified 2' position.

In one aspect, the antisense strand comprises a plurality of groups of modified nucleotides, referred to herein as "modified groups", wherein each modified group consists of one or more identically modified nucleotides, wherein each modified group is flanked on one or both sides by a second group of nucleotides, referred to herein as "flanking groups", wherein each said flanking group consists of one or more nucleotides that are either unmodified or modified in a manner different from the nucleotides of said modified group. In one embodiment, each modified group in the antisense strand is identical, i.e., each modified group consists of an equal number of identically modified nucleotides. In another embodiment, each flanking group has an equal number of nucleotides. In another embodiment, each flanking group is identical. In another embodiment, the nucleotides of said modified groups in the antisense strand comprise a modified base. In another embodiment, the nucleotides of said modified groups comprise a modified phosphate backbone. In another embodiment, the nucleotides of said modified groups comprise a modified 2' position.

In another aspect, the sense strand comprises a plurality of groups of modified groups, wherein each modified group consists of one or more identically modified nucleotides, wherein each modified group is flanked on one or both sides by a flanking group, wherein each said flanking group consists of one or more nucleotides that are either unmodified or modified in a manner different from the nucleotides of said modified group. In one embodiment, each modified group in the sense strand is identical. In another embodiment, each flanking group has an equal number of nucleotides. In another embodiment, each flanking group is identical. In another embodiment, the nucleotides of said modified groups in the sense strand comprise a modified base. In another embodiment, the nucleotides of said modified groups comprise a modified phosphate backbone. In another embodiment, the nucleotides of said modified groups comprise a modified 2' position.

In another aspect, the antisense strand and the sense strand each comprise a plurality of modified groups, wherein each modified group consists of one or more identically modified nucleotides, wherein each modified group is flanked on one or both sides by a flanking group, wherein each said flanking group consists of one or more nucleotides that are either unmodified or modified in a manner different from the nucleotides of said modified group. In one embodiment, each modified group in the antisense strand and the sense strand are identical. In another embodiment, each flanking group in the antisense strand and the sense strand each have an equal number of nucleotides. In another embodiment, each flanking group in the antisense strand and in the sense strand are identical. In another embodiment, the nucleotides of said modified groups in the antisense strand and the sense strand each comprise the same modified groups and the same flanking groups. In another embodiment, the nucleotides of said modified groups in the antisense strand and the sense strand each comprise a modified base. In another embodiment, the nucleotides of said modified groups in the antisense strand and the sense strand each comprise a modified phosphate backbone. In another embodiment, the nucleotides of said modified groups in the antisense strand and the sense strand each comprise a modified 2' position.

In another aspect, the modified groups and the flanking groups form a regular pattern on the antisense stand. In another aspect, the modified groups and the flanking groups form a regular pattern on the sense strand. In one embodiment, the modified groups and the flanking groups form a regular pattern on the both the antisense strand and the sense strand. In another embodiment, the modified groups and the flanking groups form a regular pattern on the antisense duplex region. In another aspect, the modified groups and the flanking groups form a regular pattern on the sense duplex region. In one embodiment, the modified groups and the flanking groups form a regular pattern on the both the antisense duplex region and the sense duplex region.

In another aspect, the pattern is a spatial or positional pattern. A spatial or positional pattern means that (a) nucleotide(s) are modified depending on their position within the nucleotide sequence of a double-stranded portion. Accordingly, it does not matter whether the nucleotide to be modified is a pyrimidine or a purine. Rather the position of a modified nucleotide is dependent upon: (a) its numbered position on a strand of nucleic acid, wherein the nucleotides are numbered from the 5'-end to the 3'-end with the 5'-end nucleotide of the strand being position one (both the antisense strand and sense strand are numbered from their respective 5'-end nucleotide), or (b) the position of the modified group relative to a flanking group. Thus, according to this embodiment, the modification pattern will always be the same, regardless of the sequence which is to be modified.

In one embodiment, each modified group on both the antisense strand and the sense strand is identical. In one embodiment, each modified group on both the antisense duplex region and the sense duplex region is identical. In another embodiment, each modified group and each flanking group on both the antisense strand and the sense strand are identical. In one embodiment, each modified group and each flanking group on both the antisense duplex region and the sense duplex region are identical.

In one embodiment, each modified group, each modified group position, each flanking group and each flanking group position on both the antisense strand and the sense strand are identical. In one embodiment, each modified group, each modified group position, each flanking group and each flanking group position on both the antisense duplex region and the sense duplex region are identical. In another embodiment, the modified groups on the antisense strand are complementary with the modified groups on the sense strand (the modified groups on the antisense strand and the sense strand are perfectly aligned across from one another). In another embodiment, there are no mismatches in the modified groups such that each modified group on the antisense strand is base paired with each modified group on the sense strand.

In another embodiment, each modified group on the sense strand is shifted by 1, 2, 3, 4 or 5 nucleotides relative to the modified groups on the antisense strand. For example, if each modified group on the sense strand is shifted by one nucleotide or one group of nucleotides and a modified group started at position one on the antisense strand, a modified group on the sense strand would begin at position two. In another embodiment, the modified groups of the antisense strand do not overlap the modified groups of the sense strand, i.e., no nucleotide of a modified group on the antisense strand is base paired with a nucleotide of a modified group on the sense strand.

In one embodiment, deoxyribonucleotides at an end of a strand of nucleic acid are not considered when determining a position of a modified group, i.e., the positional numbering begins with the first ribonucleotide or modified ribonucleotide. In another embodiment, abasic nucleotides at an end of a strand of nucleic acid are not considered when determining a position of a modified group.

In one aspect, a modified group comprises a 5'-end nucleotide of either or both of the antisense strand and the sense strand. In another embodiment, a flanking group comprises the 5'-end nucleotide of either or both of the antisense strand and the sense strand. In another embodiment, the 5'-end nucleotide of either or both of the antisense strand and the sense strand is unmodified. In another embodiment, a modified group comprises the 5'-end nucleotide of either or both of the antisense duplex region and sense duplex region. In another embodiment, a flanking group comprises the 5'-end nucleotide of either or both of the antisense duplex region or the sense duplex region. In another embodiment, the 5'-end nucleotide of either or both of the antisense duplex region or the sense duplex region is unmodified. In one embodiment, the modification at the 2' position is selected from the group comprising amino, fluoro, methoxy, alkoxy and C₁-C₃-alkyl. In another embodiment, the modification may be selected from 2'-O-methyl, 2'-O-methyl, 2'-O-alkyl, and 2'-O-(C₁-C₃-alkyl). In another embodiment, the modification at the 2' position is 2'-O-methyl.

### Formulations for delivery of the conjugates of the present invention

Conjugates of the present invention (such as siRNAs) can be delivered to cells, both in vitro and in vivo, by a variety of methods known to those skilled in the art, including direct contact with cells ("naked" RNA) or by combination with one or more agents that facilitate targeting or delivery into cells. Such agents and methods include lipoplexes, liposomes, iontophoresis, hydrogels, cyclodextrins, nanocapsules, micro- and nanospheres and proteinaceous vectors. The nucleic acid/vehicle combination may be locally delivered in vivo by direct injection or by use of an infusion pump. Conjugates of the invention (such as siRNAs) can be delivered in vivo by various means including intravenous subcutaneous, intramuscular or intradermal injection or inhalation. The molecules can be used as pharmaceutical agents. Preferably, pharmaceutical agents prevent, modulate the occurrence, treat or alleviate a symptom of a disease state in a subject.

Conjugates of the invention (such as siRNAs) may be formulated as pharmaceutical compositions which may further comprise a pharmaceutically acceptable carrier. As used herein the language "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

Conjugates of the invention (such as siRNAs) may be used as medicaments or as diagnostic agents, alone or in combination with other agents. For example, one or more conjugates of the present invention can be combined with a delivery vehicle (e.g., liposomes) and excipients, such as carriers, diluents. Other agents such as preservatives and stabilizers can also be added. Methods for the delivery of nucleic acid conjugates are known in the art and within the knowledge of the person skilled in the art.

Pharmaceutically acceptable compositions may comprise a therapeutically-effective amount of one or more conjugates of the invention (such as siRNAs), taken alone or formulated with one or more pharmaceutically acceptable carriers, excipient and/or diluents.

Examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) lubricating agents, such as magnesium state, sodium lauryl sulfate and talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminium hydroxide; (15) alginic acid; (16) pyrogen- free water; (17) isotonic saline; (18) Ringer's solution; (19) ethyl alcohol; (20) pH buffered solutions; (21) polyesters, polycarbonates and/or polyanhydrides; (22) bulking agents, such as polypeptides and amino acids (23) serum component, such as serum albumin, HDL and LDL; and (22) other non-toxic compatible substances employed in pharmaceutical formulations.

Stabilisers may be agents that stabilise the conjugates of the invention (such as siRNAs), for example a protein that can complex with the nucleic acid, chelators (e.g. EDTA), salts, RNAse inhibitors, and DNAse inhibitors.

In some cases it is desirable to slow the absorption of the drug from subcutaneous or intramuscular injection in order to prolong the effect of a drug. This may be accomplished by the use of a liquid suspension of crystalline or amorphous material having poor water solubility. The rate of absorption of the drug then depends upon its rate of dissolution which, in turn, may depend upon crystal size and crystalline form. Alternatively, delayed absorption of a parenterally-administered drug form is accomplished by dissolving or suspending the drug in an oil vehicle.

The conjugates of the present invention can also be administered in combination with other therapeutic compounds, either administrated separately or simultaneously, e.g., as a combined unit dose. In one embodiment, the invention includes a pharmaceutical composition comprising one or more conjugates of the present invention in a physiologically/pharmaceutically acceptable excipient, such as a stabilizer, preservative, diluent, buffer, and the like. Conjugates of the invention (such as siRNAs) may, for example be formulated in water for example water for injection, saline or phosphate buffered saline. The pharmaceutical composition may be a sterile injectable aqueous suspension or solution, or in a lyophilized form. In one embodiment, the pharmaceutical composition may comprise lyophilized lipoplexes or an aqueous suspension of lipoplexes. The lipoplexes preferably comprises a RNA of the present invention. Such lipoplexes may be used to deliver the RNA of the invention to a target cell either in vitro or in vivo.

### Surfactants

Compositions comprising the conjugates of the invention (such as siRNAs) may include a surfactant. In one embodiment, the conjugate of the invention (such as siRNAs) is formulated as an emulsion that includes a surfactant.

A surfactant that is not ionized is a non-ionic surfactant. Examples include non-ionic esters, such as ethylene glycol esters, propylene glycol esters, glyceryl esters etc., nonionic alkanolamides, and ethers such as fatty alcohol ethoxylates, propoxylated alcohols, and ethoxylated/propoxylated block polymers.

A surfactant that carries a negative charge when dissolved or dispersed in water is an anionic surfactant. Examples include carboxylates, such as soaps, acyl lactylates, acyl amides of amino acids, esters of sulfuric acid such as alkyl sulfates and ethoxylated alkyl sulfates, sulfonates such as alkyl benzene sulfonates, acyl isethionates, acyl taurates and sulfosuccinates, and phosphates.

A surfactant that carries a positive charge when dissolved or dispersed in water is a cationic surfactant. Examples include quaternary ammonium salts and ethoxylated amines.

A surfactant that has the ability to carry either a positive or negative charge is an amphoteric surfactant. Examples include acrylic acid derivatives, substituted alkylamides, N-alkylbetaines and phosphatides.

### Micelles and Other Membranous Formulations

"Micelles" are defined herein as a particular type of molecular assembly in which amphipathic molecules are arranged in a spherical structure such that all the hydrophobic portions of the molecules are directed inward, leaving the hydrophilic portions in contact with the surrounding aqueous phase. The converse arrangement exists if the environment is hydrophobic. A micelle may be formed by mixing an aqueous solution of the nucleic acid, an alkali metal alkyl sulphate, and at least one micelle forming compound.

Exemplary micelle forming compounds include lecithin, hyaluronic acid, pharmaceutically acceptable salts of hyaluronic acid, glycolic acid, lactic acid, chamomile extract, cucumber extract, oleic acid, linoleic acid, linolenic acid, monoolein, monooleates, monolaurates, borage oil, evening of primrose oil, menthol, trihydroxy oxo cholanyl glycine and pharmaceutically acceptable salts thereof, glycerin, polyglycerin, lysine, polylysine, triolein, polyoxyethylene ethers and analogues thereof, polidocanol alkyl ethers and analogues thereof, chenodeoxycholate, deoxycholate, and mixtures thereof.

Phenol and/or m-cresol may be added to the mixed micellar composition to act as a stabiliser and preservative. An isotonic agent such as glycerine may as be added.

### Particles

A composition comprising conjugate of the invention (such as siRNAs) may be incorporated into a particle such as a microparticle. Microparticles can be produced by spray-drying, lyophilisation, evaporation, fluid bed drying, vacuum drying, or a combination of these methods.

### Dosage

Dosage levels for the conjugates of the present invention can be determined by those skilled in the art by routine experimentation. In one embodiment, a unit dose may contain between about 0.01 mg/kg and about 100 mg/kg body weight of RNA. Alternatively, the dose can be from 10 mg/kg to 25 mg/kg body weight, or 1 mg/kg to 10 mg/kg body weight, or 0.05 mg/kg to 5 mg/kg body weight, or 0.1 mg/kg to 5 mg/kg body weight, or 0.1 mg/kg to1 mg/kg body weight, or 0.1 mg/kg to 0.5 mg/kg body weight, or 0.5 mg/kg to 1 mg/kg body weight.

The conjugates of the present invention may be administered to a mammalian subject in a pharmaceutically effective dose. The mammal may be selected from humans, dogs, cats, horses, cattle, pig, goat, sheep, mouse, rat, hamster and guinea pig.

In one embodiment, a subject is administered an initial dose and one or more maintenance doses of a conjugate of the invention (such as siRNAs). The maintenance dose or doses can be the same or lower than the initial dose, e.g., one-half less of the initial dose. The maintenance doses are, for example, administered no more than once every 2, 5, 10, or 30 days. The treatment regimen may last for a period of time which will vary depending upon the nature of the particular disease, its severity and the overall condition of the patient.

### Routes of Delivery

The conjugates of the present invention can be delivered to a subject by a variety of routes. Exemplary routes include: subcuteanous, intravenous, topical, rectal, anal, vaginal, nasal, pulmonary, ocular.

The conjugates of the present invention may be administered in a number of ways depending upon whether local or systemic treatment is desired and upon the area to be treated. Administration may be topical (including ophthalmic, vaginal, rectal, intranasal, transdermal), oral or parenteral. Parenteral administration includes intravenous drip, subcutaneous, intraperitoneal or intramuscular injection, or intrathecal or intraventricular administration.

The pharmaceutical composition may be specially formulated for administration in solid or liquid form. The composition may be formulated for oral administration, parenteral administration (including, for example, subcutaneous, intramuscular, intravenous, or epidural injection), topical application, intravaginal or intrarectal administration, sublingual administration, ocular administration, transdermal administration, or nasal administration. Delivery using subcutaneous or intravenous methods are preferred.

The route and site of administration may be chosen to enhance targeting. For example, to target muscle cells, intramuscular injection into the muscles of interest would be a logical choice. Lung cells might be targeted by administering the conjugate in aerosol form. The vascular endothelial cells could be targeted by coating a balloon catheter with the RNA and mechanically introducing the iRNA.

### Uses and Methods

The conjugates of the present invention may have use in medicine. In particular, the conjugates of the present invention may be used for the treatment of liver disease, genetic disease, hemophilia and bleeding disorder, liverfibrosis, non alcoholic steatohepatitis (NASH), non alcoholic fatty liver disease (NAFLD), viral hepatitis, rare diseases (e.g. acromegaly), metabolic diseases (e.g. hypercholesterolemia, dyslipidemia, hypertriglyceridemia), cardiovascular diseases, obesity, hemochromatosis, thalassemia, liver injury, alcoholic liver diseases, alcohol dependence and/or anemia of chronic disease.

In a further aspect of the invention there is provided a method of delivery of nucleic acids to hepatocytes using the conjugates according to the present invention. The method comprises the steps of contacting the hepatocyte with the compound of the present invention. The method may be used in vitro or in vivo, for diagnostic purposes, therapy or research purposes.

There is provided a method of making a conjugate of the invention the method comprising adding together the components of the conjugate to form the conjugate.

In addition, the invention provides a method of inhibiting (in vitro or in vivo) the expression of a target gene in a mammalian cell, the method comprising contacting the mammalian cell with a conjugate of the invention or a pharmaceutical composition of the invention.

There is also provided a method of inducing RNAi in a subject, the method comprising administering to the subject an effective amount of a conjugate of the invention, or a composition of the invention.

In particular, any one of the above methods may be used in the treatment of liver disease, genetic disease, hemophilia and bleeding disorder, liver fibrosis, non alcoholic steatohepatitis (NASH), non alcoholic fatty liver disease (NAFLD), viral hepatitis, rare diseases (e.g. acromegaly), metabolic diseases (e.g. hypercholesterolemia, dyslipidemia, hypertriglyceridemia), cardiovascular diseases, obesity, hemochromatosis, thalassemia, liver injury, alcoholic liver diseases, alcohol dependence and/or anemia of chronic disease in patient in need thereof.

Further aspects of the invention are defined by the following clauses:
1. A conjugate for inhibiting expression of a target gene in a cell, said conjugate comprising a nucleic acid portion and ligand portions, said nucleic acid portion comprising at least one duplex region that comprises at least a portion of a first RNA strand and at least a portion of a second RNA strand that is at least partially complementary to the first strand, wherein said first strand is at least partially complementary to at least a portion of RNA transcribed from said target gene, said ligand portions comprising a serinol-derived linker moiety and a targeting ligand for *in vivo* targeting of cells and being conjugated exclusively to the 3' and/or 5' ends of one or both RNA strands, wherein the 5' end of the first RNA strand is not conjugated, wherein:
   (i) the second RNA strand is conjugated at the 5' end to the targeting ligand, and wherein (a) the second RNA strand is also conjugated at the 3' end to the targeting ligand and the 3' end of the first RNA strand is not conjugated; or (b) the first RNA strand is conjugated at the 3' end to the targeting ligand and the 3' end of the second RNA strand is not conjugated; or (c) both the second RNA strand and the first RNA strand are also conjugated at the 3' ends to the targeting ligand; or
   (ii) both the second RNA strand and the first RNA strand are conjugated at the 3' ends to the targeting ligand and the 5' end of the second RNA strand is not conjugated.
2. The conjugate according to clause 1 wherein the second RNA strand is conjugated at the 5' end to the targeting ligand, the second RNA strand is also conjugated at the 3' end to the targeting ligand and the 3' end of the first RNA strand is not conjugated.
3. The conjugate according to clause 1 wherein the second RNA strand is conjugated at the 5' end to the targeting ligand, the first RNA strand is conjugated at the 3' end to the targeting ligand and the 3' end of the second RNA strand is not conjugated.
4. The conjugate according to clause 1 wherein the second RNA strand is conjugated at the 5' end to the targeting ligand and both the second RNA strand and the first RNA strand are also conjugated at the 3' ends to the targeting ligand.
5. The conjugate according to clause 1 wherein both the second RNA strand and the first RNA strand are conjugated at the 3' ends to the targeting ligand and the 5' end of the second RNA strand is not conjugated.
6. The conjugate according to any one of clauses 1 to 5 wherein the ligands are monomeric ligands.
7. The conjugate according to any one of clauses 1 to 6 wherein the ligands are selected from GalNAc and galactose moieties, especially GalNAc moieties.
8. The conjugate according to any one of clauses 1 to 7 wherein the conjugated RNA strands are conjugated to a targeting ligand via a serinol-derived linker moiety including a further linker wherein the further linker is or comprises a saturated, unbranched or branched C₁₋₁₅ alkyl chain, wherein optionally one or more carbons (for example 1, 2 or 3 carbons, suitably 1 or 2, in particular 1) is/are replaced by a heteroatom selected from O, N, S(O)ₚ wherein p is 0, 1 or 2, (for example a CH₂ group is replaced with O, or with NH, or with S, or with SO₂ or a -CH₃ group at the terminus of the chain or on a branch is replaced with OH or with NH₂) wherein said chain is optionally substituted by one or more oxo groups (for example 1 to 3, such as 1 group).
9. The conjugate according to clause 8 wherein the further linker comprises a saturated, unbranched C₁₋₁₅ alkyl chain wherein one or more carbons (for example 1, 2 or 3 carbons, suitably 1 or 2, in particular 1) is/are replaced by an oxygen atom.
10. The conjugate according to clause 9 wherein the further linker comprises a PEG-chain.
11. The conjugate according to clause 8 wherein the further linker comprises a saturated, unbranched C₁₋₁₅ alkyl chain.
12. The conjugate according to clause 11 wherein the further linker comprises a saturated, unbranched C₁₋₆ alkyl chain.
13. The conjugate according to clause 12 wherein the further linker comprises a saturated, unbranched C₄ or C₆ alkyl chain, e.g. a C₄ alkyl chain.
14. The conjugate according to clause 1 wherein the first RNA strand is a compound of formula (I): 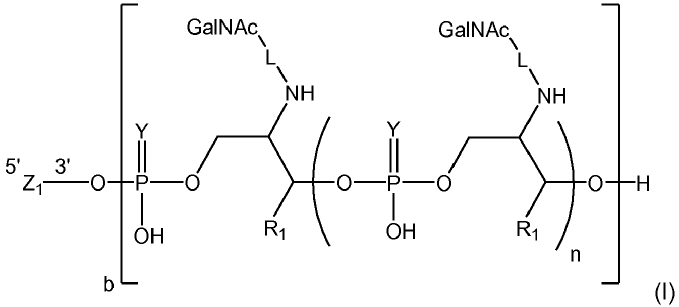 wherein b is 0 or 1; and
   the second RNA strand is a compound of formula (II): 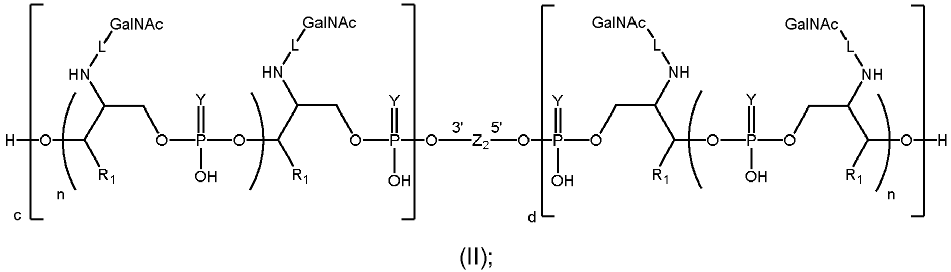 wherein c and d are independently 0 or 1;
   wherein:
   Z₁ and Z₂ are the RNA portions of the first and second RNA strands respectively;
   Y is O or S;
   R₁ is H or methyl;
   n is 0, 1, 2 or 3; and
   L is the same or different in formulae (I) and (II) and is selected from the group consisting of:
      - (CH₂)ᵣ-C(O)-, wherein r = 2-12;
      - (CH₂-CH₂-O)ₛ-CH₂-C(O)-, wherein s = 1-5;
      - (CH₂)ₜ-CO-NH-(CH₂)ₜ-NH-C(O)-, wherein t is independently is 1-5;
      - (CH₂)ᵤ-CO-NH-(CH₂)ᵤ-C(O)-, wherein u is independently is 1-5; and
      - (CH₂)ᵥ-NH-C(O)-, wherein v is 2-12; and
   wherein the terminal C(O) (if present) is attached to the NH group;
   and wherein b + c + d is 2 or 3.
15. The conjugate according to clause 14 wherein b is 0, c is 1 and d is 1.
16. The conjugate according to clause 14 wherein b is 1, c is 0 and d is 1.
17. The conjugate according to clause 14 wherein b is 1, c is 1 and d is 0.
18. The conjugate according to clause 14 wherein b is 1, c is 1 and d is 1.
19. The conjugate according to any one of clauses 14-18 wherein Y is O.
20. The conjugate according to any one of clauses 14-18 wherein Y is S.
21. The conjugate according to any one of clauses 14-20 wherein R₁ is H.
22. The conjugate according to any one of clauses 14-20 wherein R₁ is methyl.
23. The conjugate according to any one of clauses 14-22 wherein n is 0.
24. The conjugate according to any one of clauses 14-23 wherein L is -(CH₂)ᵣ-C(O)-, wherein r = 2-12.
25. The conjugate according to clause 24 wherein r = 2-6.
26. The conjugate according to clause 25 wherein r = 4 or 6 e.g. 4.
27. The conjugate according to any one of clause 1-26 wherein the targeted cells are hepatocytes.
28. The conjugate according to any one of clauses 1-27 wherein the nucleic acid portion comprises or consists of two RNA strands of 15-30 based-paired ribonucleotides, suitably 19-25 e.g. 19-23 based-paired ribonucleotides.
29. The conjugate according to any one of clauses 1-28 wherein the first RNA strand has one to six (e.g. one to three) phosphorothioate internucleotide linkages at each end.
30. The conjugate according to any one of clauses 1-29 wherein the second RNA strand has one to six (e.g. one to three) phosphorothioate internucleotide linkages at each end.
31. A method of making the conjugate, as described in any one of clauses 1-30, the method comprising adding together the components of the conjugate to form the conjugate.
32. A pharmaceutical composition comprising the conjugate according to any one of clauses 1-30 together with a pharmaceutically acceptable diluent or carrier.
33. The conjugate according to any one of clauses 1-30 or the pharmaceutical composition according to clause 32 for use in medicine.
34. The conjugate or composition, for use as described in clause 33, wherein the use for treating liver disease, genetic disease, hemophilia and bleeding disorder, liver fibrosis, non alcoholic steatohepatitis (NASH), non alcoholic fatty liver disease (NAFLD), viral hepatitis, rare diseases (e.g. acromegaly), metabolic diseases (e.g. hypercholesterolemia, dyslipidemia, hypertriglyceridemia), cardiovascular diseases, obesity, hemochromatosis, thalassemia, liver injury, alcoholic liver diseases, alcohol dependence and/or anemia of chronic disease.
35. A method of inhibiting (in vitro or in vivo) the expression of a target gene in a mammalian cell, the method comprising contacting the mammalian cell with the conjugate as defined in any one of clauses 1 to 30, or a composition as described in clause 32.
36. A method of inducing RNAi in a subject, the method comprising administering to the subject an effective amount of the conjugate as described in any one of clauses 1 to 30, or a composition as described in clause 32.
37. A method as described in clause 35 or clause 36 for use in the treatment of liver disease, genetic disease, hemophilia and bleeding disorder, liver fibrosis, non-alcoholic steatohepatitis (NASH), non-alcoholic fatty liver disease (NAFLD), viral hepatitis, rare diseases (e.g. acromegaly), metabolic diseases (e.g. hypercholesterolemia, dyslipidemia, hypertriglyceridemia), cardiovascular diseases, obesity, hemochromatosis, thalassemia, liver injury, alcoholic liver diseases, alcohol dependence and/or anemia of chronic disease in patient in need thereof.

The conjugates of the invention, in at least some embodiments, are expected to have one or more of the following advantageous properties
- good gene knock-down potency in an RNAi setting;
- good duration of action;
- good stability;
- good targeting of cells by the conjugated ligand;
- resistance to various nucleases;
- alleviation of immune response induction;
- improved circulation and tissue uptake;
- targeting cells by the conjugated ligand;
- uptake by cells without additional delivery means;
- activation of RNAi-mediated target gene down-regulation; and
- ease of manufacture.

### Experimental

### Abbreviations

- %FLP: percentage full length product
- °C: degrees centigrade
- 1H NMR: proton nuclear magnetic resonance
- A: angstrom(s)
- Ac: acetyl / acetic
- AEX-HPLC: Anion Exchange High Pressure Liquid Chromatography
- Cap: Capping
- CEP: cyanoethyl phosphoramidite
- CPG: controlled pore glass
- Da: dalton(s)
- DCM: Dichloromethane
- DEA: Diethylamine
- DIEA: Diisoethylamine
- DIPEA: Diisopropylethylamine
- DMAP: 4-dimethylaminopyridine
- DMSO: Dimethylsulphoxide
- DMT®: Dimethoxytrityl
- EDTA: ethylenediaminetetraacetic acid
- Eq: equivalent(s)
- ESI-: electrospray ionization
- Et: Ethyl
- G: gram(s)
- H: hour(s)
- HBTU: 2-(1H-benzotriazol-1-yl)-1,1,3,3-tetramethyluronium hexafluorophosphate
- HPLC: high performance liquid chromatography
- iPr: iso-propyl
- Kg: kilogram(s)
- Lcaa: long chain amino alkyl
- LCMS: liquid chromatography mass spectrometry
- M: Molar
- Me: Methyl
- Min: minute(s)
- mL: millilitre(s)
- MPEG: methylated polyethylene glycol
- MW: molecular weight
- Nm: Nanometer
- NMI: N-methylimidazole
- OD: optical density (absorption)
- OX: Oxidization
- PEG: Polyethyleneglycol
- PNA: peptide nucleic acid
- RT: room temperature
- s.c.: Subcutaneous
- TFA: trifluoroacetic acid
- THF: Tetrahydrofuran
- TLC: thin layer chromatography
- TMS: trimethylsilyl / trimethylsilane
- TTR: Transthyretin
- U: Micro
- UV: Ultraviolet
- v/v: volume / volume

### Example 1 - Synthesis of conjugates

Example compounds were synthesised according to methods described below and methods known to the person skilled in the art. Assembly of the oligonucleotide chain and linker building blocks was performed by solid phase synthesis applying phosphoramidte methodology. GalNAc conjugation was achieved by peptide bond formation of a GalNAc-carboxylic acid building block to the prior assembled and purified oligonucleotide having the necessary number of amino modified linker building blocks attached.

Oligonucleotide synthesis, deprotection and purification followed standard procedures that are known in the art.

All Oligonucleotides were synthesized on an AKTA oligopilot synthesizer using standard phosphoramidite chemistry. Commercially available solid support and 2'O-Methyl RNA phosphoramidites, 2'Fluoro, 2'Deoxy RNA phosphoramidites (all standard protection, ChemGenes, LinkTech) and commercially available 3'-Amino Modifier TFA Amino C-6 Icaa CPG 500Å (Chemgenes) were used. Per-acetylated galactose amine **8** is commercially available.

Ancillary reagents were purchased from EMP Biotech. Synthesis was performed using a 0.1 M solution of the phosphoramidite in dry acetonitrile and benzylthiotetrazole (BTT) was used as activator (0.3M in acetonitrile). Coupling time was 15 min. A Cap/OX/Cap or Cap/Thio/Cap cycle was applied (Cap: Ac₂O/NMI/Lutidine/Acetonitrile, Oxidizer: 0.1M I₂ in pyridine/H₂O). Phosphorothioates were introduced using standard commercially available thiolation reagent (EDITH, Link technologies). DMT cleavage was achieved by treatment with 3% dichloroacetic acid in toluene. Upon completion of the programmed synthesis cycles a diethylamine (DEA) wash was performed. All oligonucleotides were synthesized in DMT-off mode.

Attachment of the serinol-derived linker moiety was achieved by use of either base-loaded (S)-DMT-Serinol(TFA)-succinate-lcaa-CPG **10** or a (S)-DMT-Serinol(TFA) phosphoramidite **7** (synthesis was performed as described in Hoevelmann *et al.* (2016)). Tri-antennary GalNAc clusters (ST23/C4XLT) were introduced by successive coupling of the respective trebler amidite derivatives (C4XLT-phos) followed by the GalNAc amidite (ST23-phos).

The single strands were cleaved off the CPG by 40% aq. methylamine treatment. The resulting crude oligonucleotide was purified by ion exchange chromatography (Resource Q, 6mL, GE Healthcare) on a AKTA Pure HPLC System using a sodium chloride gradient. Product containing fractions were pooled, desalted on a size exclusion column (Zetadex, EMP Biotech) and lyophilised.

Individual single strands were dissolved in a concentration of 60 OD/mL in H₂O. Both individual oligonucleotide solutions were added together in a reaction vessel. For easier reaction monitoring a titration was performed. The first strand was added in 25% excess over the second strand as determined by UV-absorption at 260nm. The reaction mixture was heated to 80°C for 5min and then slowly cooled to RT. Double strand formation was monitored by ion pairing reverse phase HPLC. From the UV-area of the residual single strand the needed amount of the second strand was calculated and added to the reaction mixture. The reaction was heated to 80°C again and slowly cooled to RT. This procedure was repeated until less than 10% of residual single strand was detected.

### Synthesis of compounds 2-10

Compounds **2** to **5** and (S)-DMT-Serinol(TFA)-phosphoramidite **7** were synthesised according to literature published methods (Hoevelmann et al. Chem. Sci., 2016,7, 128-135).

### (S)-4-(3-(bis(4-methoxyphenyl)(phenyl)methoxy)-2-(2,2,2-trifluoroacetamido)propoxy)-4-oxobutanoic acid (6).

To a solution of **5** in pyridine was added succinic anhydride, followed by DMAP. The resulting mixture was stirred at room temperature overnight. All starting material was consumed, as judged by TLC. The reaction was concentrated. The crude material was chromatographed in silica gel using a gradient 0% to 5% methanol in DCM (+ 1% triethylamine) to afford 1.33 g of **6** (yield = 38%). m/z (ESI-): 588.2 (100%), (calcd. for C30H29F3NO8⁻ [M-H]⁻ 588.6). 1 H-NMR: (400 MHz, CDCl3) δ [ppm] = 7.94 (d, 1H, NH), 7.39 - 7.36 (m, 2H, CHaryl), 7.29 - 7.25 (m, 7H, CHaryl), 6.82-6.79 (m, 4H, CHaryl), 4.51 - 4.47 (m, 1H), 4.31 - 4.24 (m, 2H), 3.77 (s, 6H, 2xDMTr-OMe), 3.66 - 3.60 (m, 16H, HNEt₃⁺), 3.26 - 3.25 (m, 2H), 2.97 - 2.81 (m, 20H, NEt₃), 2.50-2.41 (4H, m), 1.48 - 1.45 (m, 26H, HNEt₃⁺), 1.24 -1.18 (m, 29H, NEt₃).

### (S)-DMT-Serinol(TFA)-succinate-lcaa-CPG (10)

The (S)-DMT-Serinol(TFA)-succinate (159 mg, 270 umol) and HBTU (113 mg, 299 umol) were dissolved in CH₃CN (10 mL). Diisopropylethylamine (DIPEA, 94 µL, 540 umol) was added to the solution, and the mixture was swirled for 2 min followed by addition native amino-Icaa-CPG (500 A, 3 g, amine content: 136 umol/g). The suspension was gently shaken at room temperature on a wrist-action shaker for 16h then filtered, and washed with DCM and EtOH. The solid support was dried under vacuum for 2 h. The unreacted amines on the support were capped by stirring with acetic anhydride/lutidine/N-methylimidazole at room temperature. The washing of the support was repeated as above. The solid was dried under vacuum to yield solid support **10** (3 g, 26 umol/g loading).

### GalNAc Synthon (9)

Synthesis of the GalNAc synthon **9** was performed as described in Nair et al. J. Am. Chem. Soc., 2014, 136 (49), pp 16958-16961, in 46% yield over two steps.

The characterising data matched the published data.

### Synthesis of Oligonucleotides

All single stranded oligonucleotides were synthesised according to the reaction conditions described above and in Figure 8 and 9.

All final single stranded products were analysed by AEX-HPLC to prove their purity. Purity is given in %FLP (% full length product) which is the percentage of the UV-area under the assigned product signal in the UV-trace of the AEX-HPLC analysis of the final product. Identity of the respective single stranded products (non-modified, amino-modified precursors or GalNAc conjugated oligonucleotides) was proved by LC-MS analysis.

**Table 2: Single stranded un-conjugated oligonucleotides**

| **Product (11)** | **Name** | **MW calc.** | **MW (ESI-) found** | **%FLP (AEX-HPLC)** |
|---|---|---|---|---|
| A0002 | STS16001A | 6943.3 Da | 6943.0 Da | 86.6% |
| A0006 | STS16001BL4 | 8387.5 Da | 8387.5 Da | 94.1% |
| A0130 | STS18001A | 6259.9 Da | 6259.8 Da | 76.5% |
| A0131 | STS18001BL4 | 7813.2 Da | 7813.1 Da | 74.3% |
| A0220 | STS16001B-5'1xNH2 | 6982.2 Da | 6982.1 Da | 95.7% |
| A0237 | STS16001A | 6943.3 Da | 6943.3 Da | 95.6% |
| A0244 | STS16001BV1 | 6845.2 Da | 6844.9 Da | 98.2% |
| A0264 | STS16001AV4-3'1xNH2 | 7112.4 Da | 7112.2 Da | 95.4% |
| A0329 | STS16001BV6-3'5'1xNH2 | 7183.3 Da | 7183.2 Da | 88.8% |

5'1 x NH2 means refers to the position (5' end) and number (1 x NH2) of free serinol derived amino groups which are available for conjugation. For example, 1x3'NH2 on **A0264** means there is free amino group which can be reacted with GalNAc synthon **9** at the 3' end of the strand **A0264.** 3'5'1xNH2 means there is one serinol-derived free amino group which can be reacted with GalNAc linker **9** at the 3' end and the 5' end of the strand.

### Synthesis of conjugates 1-3 and reference conjugates 1-2

### Conjugated singles strands for conjugates 1-2 and reference conjugates 1-2

Conjugation of the GalNac synthon (**9**) was achieved by coupling to the serinol-amino function of the respective oligonucleotide strand **11** using a peptide coupling reagent. Therefore, the respective amino-modified precursor molecule **11** was dissolved in H₂O (500 OD/mL) and DMSO (DMSO/H₂O, 2/1, v/v) was added, followed by DIPEA (2.5% of total volume). In a separate reaction vessel pre-activation of the GalN(Ac4)-C4-acid (**9**) was performed by reacting 2 eq. (per amino function in the amino-modified precursor oligonucleotide **11**) of the carboxylic acid component with 2 eq. of HBTU in presence of 8 eq. DIPEA in DMSO. After 2 min the pre-activated compound **9** was added to the solution of the respective amino-modified precursor molecule. After 30 min the reaction progress was monitored by LCMS or AEX-HPLC. Upon completion of the conjugation reaction the crude product was precipitated by addition of 10x *i*PrOH and 0.1x 2M NaCl and harvested by centrifugation and decantation. To set free the acetylated hydroxyl groups in the GalNAc moieties the resulting pellet was dissolved in 40% MeNH2 (1mL per 500 OD) and after 15 min at RT diluted in H₂O (1:10) and finally purified again by anion exchange and size exclusion chromatography and lyophilised to yield the final product **12.**

**Table 3: Single stranded GalNAc-conjugated oligonucleotides**

| **Product (12)** | **Starting Material** | **Name** | **MW calc.** | **MW (ESI-) found** | **%FLP (AEX-HPLC)** |
|---|---|---|---|---|---|
| A0241 | A0220 | STS16001BL20 | 7285.5 Da | 7285.3 Da | 91.8% |
| A0268 | A0264 | STS16001AV4L33 | 7415.7 Da | 7415.4 Da | 96.9% |
| A0330 | A0329 | STS16001BV6L42 | 7789.8 Da | 7789.8 Da | 95.5% |

### Double strand formation

Double strand formation was performed according to the methods described above.

The double strand purity is given in % double strand which is the percentage of the UV-area under the assigned product signal in the UV-trace of the IP-RP-HPLC analysis.

**Table 4: Nucleic acid conjugates**

| **Product** | **Starting Materials** | | **Name** | **% double strand** |
|---|---|---|---|---|
| | **First Strand** | **Second Strand** | | |
| Ref. Conj. 1 | A0237 | A0241 | STS16001L20 | 97.7% |
| Ref. Conj. 2 | A0268 | A0244 | STS16001L33 | 97.8% |
| Ref. Conj. 3 | A0130 | A0131 | STS18001L4 | 96.8% |
| Ref. Conj. 4 | A0002 | A0006 | STS16001L4 | 90.1% |
| Conjugate 1 | A0268 | A0241 | STS16001L24 | 96.0% |
| Conjugate 2 | A0237 | A0330 | STS16001V1L42 | 98.5% |
| Conjugate 3 | A0268 | A0330 | STS16001V1L43 | 98.2% |

### Sequences

Modifications key for the following sequences:
f denotes 2'Fluoro 2'deoxyribonucleotide or 2'-fluoro ribonucleotide (the terms are interchangeable)
m denotes 2'O Methyl ribonucleotide
(ps) denotes phosphorothioate linkage
Ser(GN) is a GalNAc-C4 building block attached to serinol derived linker moiety: 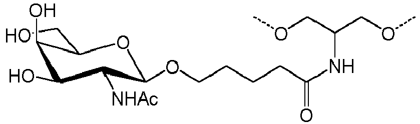
wherein the O--- is the linkage between the oxygen atom and e.g. H, phosphordiester linkage or phosphorothioate linkage.

C4XLT is:

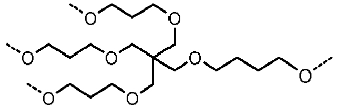

ST23 is:

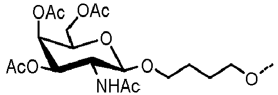

Synthesis of the phosphoramidite derivatives of C4XLT (C4XLT-phos) as well as ST23 (ST23-phos) can be performed as described in WO2017/174657.

C4XLT-phos:

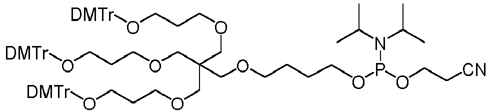

ST23-phos:

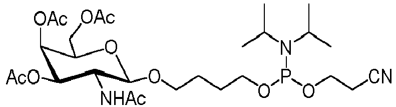

### Conjugate 1

Antisense strand - STS16001AL33
   5' mU (ps) fU (ps) mA fU mA fG mA fG mC fA mA fG mA fA mC fA mC fU mG (ps) fU (ps) mU (ps) Ser(GN) 3'
Sense strand - STS16001BL20
   5' Ser(GN) (ps) fA mA fC mA fG mU fG mU fU mC fU mU fG mC fU mC fU mA fU (ps) mA (ps) fA 3'

### Conjugate 2

Antisense strand - STS16001A
   mU (ps) fU (ps) mA fU mA fG mA fG mC fA mA fG mA fA mC fA mC fU mG (ps) fU (ps) mU
Sense strand - STS16001BV1L42
   Ser(GN) (ps) fA (ps) mA (ps) fC mA fG mU fG mU fU mC fU mU fG mC fU mC fU mA fU (ps) mA (ps) fA (ps) Ser(GN)

### Conjugate 3

Antisense strand - STS16001AL33
   5' mU (ps) fU (ps) mA fU mA fG mA fG mC fA mA fG mA fA mC fA mC fU mG (ps) fU (ps) mU (ps) Ser(GN) 3'
Sense strand - STS16001BV1L42
   5' Ser(GN) (ps) fA (ps) mA (ps) fC mA fG mU fG mU fU mC fU mU fG mC fU mC fU mA fU (ps) mA (ps) fA (ps) Ser(GN) 3'

### Reference conjugate 1

Antisense strand - STS16001A
   mU (ps) fU (ps) mA fU mA fG mA fG mC fA mA fG mA fA mC fA mC fU mG (ps) fU (ps) mU
Sense strand - STS16001BL20
   Ser(GN) (ps) fA mA fC mA fG mU fG mU fU mC fU mU fG mC fU mC fU mA fU (ps) mA (ps) fA

### Reference conjugate 2

Antisense strand - STS16001AL33
   mU (ps) fU (ps) mA fU mA fG mA fG mC fA mA fG mA fA mC fA mC fU mG (ps) fU (ps) mU (ps) Ser(GN)
Sense strand - STS16001BV1
   fA (ps) mA (ps) fC mA fG mU fG mU fU mC fU mU fG mC fU mC fU mA fU (ps) mA (ps) fA

### Reference Conjugate 3

Antisense strand - STS18001A (A0130)
   mU (ps) fC (ps) mG fA mA fG mU fA mU fU mC fC mG fC mG fU mA (ps) fC (ps) mG
Sense strand - STS18001BL4 (A0131)
   [(ST23) (ps)]₃ C4XLT (ps) fC mG fU mA fC mG fC mG fG mA fA mU fA mC fU mU fC (ps) mG (ps) fA

### Reference Conjugate 4

Antisense strand - STS16001AL33
   mU (ps) fU (ps) mA fU mA fG mA fG mC fA mA fG mA fA mC fA mC fU mG (ps) fU (ps) mU
Sense strand - STS16001BL4
   5'[(ST23) (ps)]₃ C4XLT(ps)fA (ps) mA (ps) fC mA fG mU fG mU fU mC fU mU fG mC fU mC fU mA fU (ps) mA (ps) fA

### Example 2 - In vitro determination of TTR knockdown of various TTR siRNA GalNAc conjugates

Murine primary hepatocytes were seeded into collagen pre-coated 96 well plates (Thermo Fisher Scientific, #A1142803) at a cell density of 30,000 cells per well and treated with siRNA-conjugates at concentrations ranging from 10nM to 0.0001nM. 24h post treatment cells were lysed and RNA extracted with InviTrap® RNA Cell HTS 96 Kit / C24 x 96 preps (Stratec #7061300400) according to the manufactures protocol. Transcripts levels of TTR and housekeeping mRNA (Ptenll) were quantified by TaqMan analysis.

Target gene expression in primary murine hepatocytes 24h following treatment with the conjugates of the invention, Conjugates 1-3, showed that target gene expression decreases as the dose of the conjugate increased compared to the negative controls (see "UT" column and Reference Conjugate 3), as shown in Figure 10. This indicates that the first strand is binding to the target gene, thus lowering gene expression. Figure 10 also shows the target gene expression levels of Reference Conjugates 1 and 2 which act as comparator conjugates. As can be seen from a comparison between the data presented in Figures 10A and 10C, and 10B and 10C, the conjugates of the invention (Conjugates 1-3) decrease the target gene expression compared to Reference Conjugates 1 and 2. The most effective conjugate at 0.01 nM appears to be Conjugate 2. The most effective conjugate at 0.1 nM, 0.5 nM, 1 nM and 10 nM appears to be Conjugate 3.

### Example 3 - In vivo time course of serum TTR in mice

C57BL/6 mice were treated s.c. with 1mg/kg siRNA-conjugates at day 0. Serum samples were taken at day 7, 14, and 27 by orbital sinus bleeding and stored at -20°C until analysis. Serum TTR quantification was performed with a Mouse Prealbumin ELISA (ALPCO, 41-PALMS/lot 22, 2008003B) according to the manufacturers protocol (sample dilution 1:8000 or 1:800).

The results of the time course of serum TTR in c57BL/6 mice cohorts of n=4 at 7, 14, and 27 days post s.c. treatment with 1mg/kg Conjugates 1-3, Reference Conjugates 1, 2 and 4, and mock treated (PBS) individuals is shown in Figure 11. As indicated by the data in Figure 11, the conjugates of the invention are particularly effective at reducing target gene expression compared to the negative control (PBS) and Reference Conjugates 1, 2, and in particular to Reference Conjugate 4. Conjugates 2 and 3 are also more effective than Reference Conjugates 1, 2 and 4. The most effective conjugate is Conjugate 2. Thus, it may be expected that the dosing level of Conjugate 3 would be about three times lower to achieve the same initial knock down and would also result in longer duration of knock down as compared to Reference Conjugate 4.

All patents and patent applications referred to herein are incorporated by reference in their entirety.

Throughout the specification and the claims which follow, unless the context requires otherwise, the word 'comprise', and variations such as 'comprises' and 'comprising', will be understood to imply the inclusion of a stated integer, step, group of integers or group of steps but not to the exclusion of any other integer, step, group of integers or group of steps.

### References

1. Fire, A.; Xu, S.; Montgomery, M. K.; Kostas, S. A.; Driver, S. E.; Mello, C. C., Potent and specific genetic interference by double-stranded RNA in Caenorhabditis elegans. Nature 1998, 391 (6669), 806-11.
2. Elbashir, S. M.; Lendeckel, W.; Tuschl, T., RNA interference is mediated by 21- and 22-nucleotide RNAs. Genes & development 2001, 15 (2), 188-200.
3. Matsuda et al (2015) ACS Chem. Biol. 10, 1181-1187
4. Schmidt et al (2017) Nucl. Acids Res. 45(5) 2294-2306
5. Kamiya et al (2014) ChemBioChem 15, 2549-2555
6. Nair, J. K.; Willoughby, J. L. S.; Chan, A., Charisse, K., Alam, Md. R.; Wang, Q.; Hoekstra, M.; Kandasamy, P.; Kel'in, A. V.; Milstein, S.; Taneja, N.; O'Shea, J.; Shaikh, S.; Zhang, L.; van der Sluis, R. J.; Jung, M. E.; Akinc, A.; Hutabarat, R.; Kuchimanchi, S.; Fitzgerald, K.; Zimmermann, T.; van Berkel, T. J. C.; Maier, M. A.; Rajeev, K. G.; and Manoharan, M., Multivalent N-Acetylgalactosamine-Conjugated siRNA Localizes in Hepatocytes and Elicits Robust RNAi-Mediated Gene Silencing. Journal of the American Chemical Society 2014, 136 (49), 6958-16961.
7. Hövelmann, F.; Gaspar, I.; Chamiolo, J.; Kasper, M.; Steffen, J.; Ephrussi, A.; Seitz, O., LNA-enhanced DNA FIT-probes for multicolour RNA imaging. Chemical Science 2016, 7 (1), 128-135.

## Claims

1. A conjugate for inhibiting expression of a target gene in a cell, said conjugate comprising a nucleic acid portion and ligand portions, said nucleic acid portion comprising at least one duplex region that comprises at least a portion of a first RNA strand and at least a portion of a second RNA strand that is at least partially complementary to the first strand, wherein said first strand is at least partially complementary to at least a portion of RNA transcribed from said target gene, said ligand portions comprising a serinol-derived linker moiety and a targeting ligand for *in vivo* targeting of cells and being conjugated exclusively to the 3' and/or 5' ends of one or both RNA strands, wherein the 5' end of the first RNA strand is not conjugated, wherein:
(iii) the second RNA strand is conjugated at the 5' end to the targeting ligand, and wherein (a) the second RNA strand is also conjugated at the 3' end to the targeting ligand and the 3' end of the first RNA strand is not conjugated; or (b) the first RNA strand is conjugated at the 3' end to the targeting ligand and the 3' end of the second RNA strand is not conjugated; or (c) both the second RNA strand and the first RNA strand are also conjugated at the 3' ends to the targeting ligand; or
(iv) both the second RNA strand and the first RNA strand are conjugated at the 3' ends to the targeting ligand and the 5' end of the second RNA strand is not conjugated.

2. The conjugate according to claim 1 wherein the second RNA strand is conjugated at the 5' end to the targeting ligand, the second RNA strand is also conjugated at the 3' end to the targeting ligand and the 3' end of the first RNA strand is not conjugated; or the second RNA strand is conjugated at the 5' end to the targeting ligand, the first RNA strand is conjugated at the 3' end to the targeting ligand and the 3' end of the second RNA strand is not conjugated; or the second RNA strand is conjugated at the 5' end to the targeting ligand and both the second RNA strand and the first RNA strand are also conjugated at the 3' ends to the targeting ligand; or both the second RNA strand and the first RNA strand are conjugated at the 3' ends to the targeting ligand and the 5' end of the second RNA strand is not conjugated.

3. The conjugate according to claim 1 or claim 2 wherein the ligands are monomeric ligands.

4. The conjugate according to any one of claims 1 to 3 wherein the ligands are selected from GalNAc and galactose moieties, especially GalNAc moieties.

5. The conjugate according to any one of claims 1 to 4 wherein the conjugated RNA strands are conjugated to a targeting ligand via a serinol-derived linker moiety including a further linker wherein the further linker is or comprises a saturated, unbranched or branched C₁₋₁₅ alkyl chain, wherein optionally one or more carbons (for example 1, 2 or 3 carbons, suitably 1 or 2, in particular 1) is/are replaced by a heteroatom selected from O, N, S(O)ₚ wherein p is 0, 1 or 2, (for example a CH₂ group is replaced with O, or with NH, or with S, or with SO₂ or a -CH₃ group at the terminus of the chain or on a branch is replaced with OH or with NH₂) wherein said chain is optionally substituted by one or more oxo groups (for example 1 to 3, such as 1 group).

6. The conjugate according to claim 5 wherein the further linker comprises a saturated, unbranched C₁₋₁₅ alkyl chain wherein one or more carbons (for example 1, 2 or 3 carbons, suitably 1 or 2, in particular 1) is/are replaced by an oxygen atom.

7. The conjugate according to claim 1 wherein the first RNA strand is a compound of formula (I): 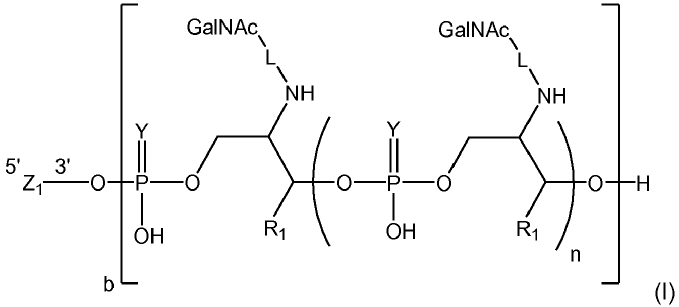 wherein b is 0 or 1; and
the second RNA strand is a compound of formula (II): 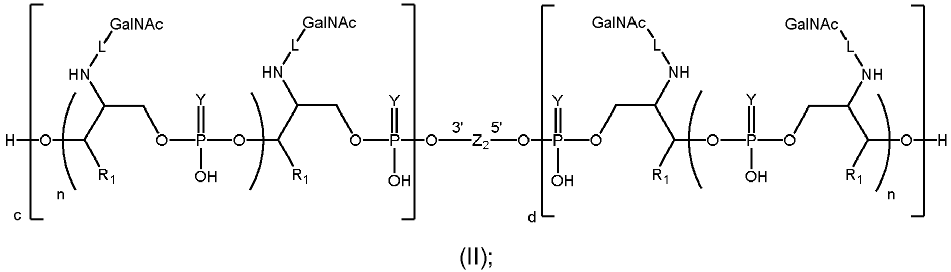 wherein c and d are independently 0 or 1;
wherein:
Z₁ and Z₂ are the RNA portions of the first and second RNA strands respectively;
Y is O or S;
R₁ is H or methyl;
n is 0, 1, 2 or 3; and
L is the same or different in formulae (I) and (II) and is selected from the group consisting of:
- (CH₂)ᵣ-C(O)-, wherein r = 2-12;
- (CH₂-CH₂-O)ₛ-CH₂-C(O)-, wherein s = 1-5;
- (CH₂)ₜ-CO-NH-(CH₂)ₜ-NH-C(O)-, wherein t is independently is 1-5;
- (CH₂)ᵤ-CO-NH-(CH₂)ᵤ-C(O)-, wherein u is independently is 1-5; and
- (CH₂)ᵥ-NH-C(O)-, wherein v is 2-12; and
wherein the terminal C(O) (if present) is attached to the NH group;
and wherein b + c + d is 2 or 3.

8. The conjugate according to claim 7 wherein b is 0, c is 1 and d is 1 or
wherein b is 1, c is 0 and d is 1; or wherein b is 1, c is 1 and d is 0, or
wherein b is 1, c is 1 and d is 1.

9. The conjugate according to claim 7 or claim 8 wherein Y is O.

10. The conjugate according to claim 7 or claim 8 wherein Y is S.

11. The conjugate according to any one of claims 7-10 wherein R₁ is H.

12. The conjugate according to any one of claims 7-10 wherein R₁ is methyl.

13. The conjugate according to any one of claims 7-12 wherein n is 0.

14. The conjugate according to any one of claims 7-13 wherein L is -(CH₂)ᵣ-C(O)-, wherein r = 2-12.

15. The conjugate according to claim 14 wherein r = 2-6.


SEQUENCE LISTING

<110>  Silence Therapeutics Plc

<120>  NUCLEIC ACID CONJUGATES

<130>  SIL-P2320EPp

<160>  8

<170>  PatentIn version 3.5

<210>  1
<211>  21
<212>  RNA
<213>  Artificial

<220>
<223>  Synthetic sequence


<220>
<221>  misc_binding
<222>  (1)..(3)
<223>  Phosphorothioate linkage between each ribonucleotide

<220>
<221>  misc_feature
<222>  (1)..(1)
<223>  2'O Methyl ribonucleotide

<220>
<221>  misc_feature
<222>  (2)..(2)
<223>  2'Fluoro 2'deoxyribonucleotide

<220>
<221>  misc_feature
<222>  (3)..(3)
<223>  2'O Methyl ribonucleotide

<220>
<221>  misc_feature
<222>  (4)..(4)
<223>  2'Fluoro 2'deoxyribonucleotide

<220>
<221>  misc_feature
<222>  (5)..(5)
<223>  2'O Methyl ribonucleotide

<220>
<221>  misc_feature
<222>  (6)..(6)
<223>  2'Fluoro 2'deoxyribonucleotide

<220>
<221>  misc_feature
<222>  (7)..(7)
<223>  2'O Methyl ribonucleotide

<220>
<221>  misc_feature

<222>  (8)..(8)
<223>  2'Fluoro 2'deoxyribonucleotide

<220>
<221>  misc_feature
<222>  (9)..(9)
<223>  2'O Methyl ribonucleotide

<220>
<221>  misc_feature
<222>  (10)..(10)
<223>  2'Fluoro 2'deoxyribonucleotide

<220>
<221>  misc_feature
<222>  (11)..(11)
<223>  2'O Methyl ribonucleotide

<220>
<221>  misc_feature
<222>  (12)..(12)
<223>  2'Fluoro 2'deoxyribonucleotide

<220>
<221>  misc_feature
<222>  (13)..(13)
<223>  2'O Methyl ribonucleotide

<220>
<221>  misc_feature
<222>  (14)..(14)
<223>  2'Fluoro 2'deoxyribonucleotide

<220>
<221>  misc_feature
<222>  (15)..(15)
<223>  2'O Methyl ribonucleotide

<220>
<221>  misc_feature
<222>  (16)..(16)
<223>  2'Fluoro 2'deoxyribonucleotide

<220>
<221>  misc_feature
<222>  (17)..(17)
<223>  2'O Methyl ribonucleotide

<220>
<221>  misc_feature
<222>  (18)..(18)
<223>  2'Fluoro 2'deoxyribonucleotide

<220>
<221>  misc_feature
<222>  (19)..(19)
<223>  2'O Methyl ribonucleotide

<220>
<221>  misc_binding
<222>  (19)..(21)
<223>  Phosphorothioate linkage between each ribonucleotide

<220>
<221>  misc_feature
<222>  (20)..(20)
<223>  2'Fluoro 2'deoxyribonucleotide

<220>
<221>  misc_feature
<222>  (21)..(21)
<223>  2'O Methyl ribonucleotide

<220>
<221>  misc_binding
<222>  (21)..(21)
<223>  Attached to Ser(GN) via a phosphorothioate linkage

<400>  1
uuauagagca agaacacugu u                                                21


<210>  2
<211>  21
<212>  RNA
<213>  Artificial

<220>
<223>  Synthetic sequence


<220>
<221>  misc_binding
<222>  (1)..(1)
<223>  Attached to Ser(GN) via a phosphorothioate linkage

<220>
<221>  misc_feature
<222>  (1)..(1)
<223>  2'Fluoro 2'deoxyribonucleotide

<220>
<221>  misc_feature
<222>  (2)..(2)
<223>  2'O Methyl ribonucleotide

<220>
<221>  misc_feature
<222>  (3)..(3)
<223>  2'Fluoro 2'deoxyribonucleotide

<220>
<221>  misc_feature
<222>  (4)..(4)
<223>  2'O Methyl ribonucleotide

<220>
<221>  misc_feature
<222>  (5)..(5)
<223>  2'Fluoro 2'deoxyribonucleotide

<220>
<221>  misc_feature
<222>  (6)..(6)
<223>  2'O Methyl ribonucleotide

<220>
<221>  misc_feature
<222>  (7)..(7)
<223>  2'Fluoro 2'deoxyribonucleotide

<220>
<221>  misc_feature
<222>  (8)..(8)
<223>  2'O Methyl ribonucleotide

<220>
<221>  misc_feature
<222>  (9)..(9)
<223>  2'Fluoro 2'deoxyribonucleotide

<220>
<221>  misc_feature
<222>  (10)..(10)
<223>  2'O Methyl ribonucleotide

<220>
<221>  misc_feature
<222>  (11)..(11)
<223>  2'Fluoro 2'deoxyribonucleotide

<220>
<221>  misc_feature
<222>  (12)..(12)
<223>  2'O Methyl ribonucleotide

<220>
<221>  misc_feature
<222>  (13)..(13)
<223>  2'Fluoro 2'deoxyribonucleotide

<220>
<221>  misc_feature
<222>  (14)..(14)
<223>  2'O Methyl ribonucleotide

<220>
<221>  misc_feature
<222>  (15)..(15)
<223>  2'Fluoro 2'deoxyribonucleotide

<220>
<221>  misc_feature
<222>  (16)..(16)
<223>  2'O Methyl ribonucleotide

<220>
<221>  misc_feature
<222>  (17)..(17)
<223>  2'Fluoro 2'deoxyribonucleotide

<220>
<221>  misc_feature
<222>  (18)..(18)
<223>  2'O Methyl ribonucleotide

<220>
<221>  misc_feature
<222>  (19)..(19)

<223>  2'Fluoro 2'deoxyribonucleotide

<220>
<221>  misc_binding
<222>  (19)..(21)
<223>  Phosphorothioate linkage between each ribonucleotide

<220>
<221>  misc_feature
<222>  (20)..(20)
<223>  2'O Methyl ribonucleotide

<220>
<221>  misc_feature
<222>  (21)..(21)
<223>  2'Fluoro 2'deoxyribonucleotide

<400>  2
aacaguguuc uugcucuaua a                                                21


<210>  3
<211>  21
<212>  RNA
<213>  Artificial

<220>
<223>  Synthetic sequence


<220>
<221>  misc_binding
<222>  (1)..(3)
<223>  Phosphorothioate linkage between each ribonucleotide

<220>
<221>  misc_feature
<222>  (1)..(1)
<223>  2'O Methyl ribonucleotide

<220>
<221>  misc_feature
<222>  (2)..(2)
<223>  2'Fluoro 2'deoxyribonucleotide

<220>
<221>  misc_feature
<222>  (3)..(3)
<223>  2'O Methyl ribonucleotide

<220>
<221>  misc_feature
<222>  (4)..(4)
<223>  2'Fluoro 2'deoxyribonucleotide

<220>
<221>  misc_feature
<222>  (5)..(5)
<223>  2'O Methyl ribonucleotide

<220>
<221>  misc_feature
<222>  (6)..(6)

<223>  2'Fluoro 2'deoxyribonucleotide

<220>
<221>  misc_feature
<222>  (7)..(7)
<223>  2'O Methyl ribonucleotide

<220>
<221>  misc_feature
<222>  (8)..(8)
<223>  2'Fluoro 2'deoxyribonucleotide

<220>
<221>  misc_feature
<222>  (9)..(9)
<223>  2'O Methyl ribonucleotide

<220>
<221>  misc_feature
<222>  (10)..(10)
<223>  2'Fluoro 2'deoxyribonucleotide

<220>
<221>  misc_feature
<222>  (11)..(11)
<223>  2'O Methyl ribonucleotide

<220>
<221>  misc_feature
<222>  (12)..(12)
<223>  2'Fluoro 2'deoxyribonucleotide

<220>
<221>  misc_feature
<222>  (13)..(13)
<223>  2'O Methyl ribonucleotide

<220>
<221>  misc_feature
<222>  (14)..(14)
<223>  2'Fluoro 2'deoxyribonucleotide

<220>
<221>  misc_feature
<222>  (15)..(15)
<223>  2'O Methyl ribonucleotide

<220>
<221>  misc_feature
<222>  (16)..(16)
<223>  2'Fluoro 2'deoxyribonucleotide

<220>
<221>  misc_feature
<222>  (17)..(17)
<223>  2'O Methyl ribonucleotide

<220>
<221>  misc_feature
<222>  (18)..(18)
<223>  2'Fluoro 2'deoxyribonucleotide

<220>

<221>  misc_binding
<222>  (19)..(21)
<223>  Phosphorothioate linkage between each ribonucleotide

<220>
<221>  misc_feature
<222>  (19)..(19)
<223>  2'O Methyl ribonucleotide

<220>
<221>  misc_feature
<222>  (20)..(20)
<223>  2'Fluoro 2'deoxyribonucleotide

<220>
<221>  misc_feature
<222>  (21)..(21)
<223>  2'O Methyl ribonucleotide

<400>  3
uuauagagca agaacacugu u                                                21


<210>  4
<211>  21
<212>  RNA
<213>  Artificial

<220>
<223>  Synthetic sequence


<220>
<221>  misc_binding
<222>  (1)..(1)
<223>  Attached to Ser(GN) via a phosphorothioate linkage

<220>
<221>  misc_binding
<222>  (1)..(3)
<223>  Phosphorothioate linkage between each ribonucleotide

<220>
<221>  misc_feature
<222>  (1)..(1)
<223>  2'Fluoro 2'deoxyribonucleotide

<220>
<221>  misc_feature
<222>  (2)..(2)
<223>  2'O Methyl ribonucleotide

<220>
<221>  misc_feature
<222>  (3)..(3)
<223>  2'Fluoro 2'deoxyribonucleotide

<220>
<221>  misc_feature
<222>  (4)..(4)
<223>  2'O Methyl ribonucleotide

<220>

<221>  misc_feature
<222>  (5)..(5)
<223>  2'Fluoro 2'deoxyribonucleotide

<220>
<221>  misc_feature
<222>  (6)..(6)
<223>  2'O Methyl ribonucleotide

<220>
<221>  misc_feature
<222>  (7)..(7)
<223>  2'Fluoro 2'deoxyribonucleotide

<220>
<221>  misc_feature
<222>  (8)..(8)
<223>  2'O Methyl ribonucleotide

<220>
<221>  misc_feature
<222>  (9)..(9)
<223>  2'Fluoro 2'deoxyribonucleotide

<220>
<221>  misc_feature
<222>  (10)..(10)
<223>  2'O Methyl ribonucleotide

<220>
<221>  misc_feature
<222>  (11)..(11)
<223>  2'Fluoro 2'deoxyribonucleotide

<220>
<221>  misc_feature
<222>  (12)..(12)
<223>  2'O Methyl ribonucleotide

<220>
<221>  misc_feature
<222>  (13)..(13)
<223>  2'Fluoro 2'deoxyribonucleotide

<220>
<221>  misc_feature
<222>  (14)..(14)
<223>  2'O Methyl ribonucleotide

<220>
<221>  misc_feature
<222>  (15)..(15)
<223>  2'Fluoro 2'deoxyribonucleotide

<220>
<221>  misc_feature
<222>  (16)..(16)
<223>  2'O Methyl ribonucleotide

<220>
<221>  misc_feature
<222>  (17)..(17)
<223>  2'Fluoro 2'deoxyribonucleotide

<220>
<221>  misc_feature
<222>  (18)..(18)
<223>  2'O Methyl ribonucleotide

<220>
<221>  misc_feature
<222>  (19)..(19)
<223>  2'Fluoro 2'deoxyribonucleotide

<220>
<221>  misc_binding
<222>  (19)..(21)
<223>  Phosphorothioate linkage between each ribonucleotide

<220>
<221>  misc_feature
<222>  (20)..(20)
<223>  2'O Methyl ribonucleotide

<220>
<221>  misc_feature
<222>  (21)..(21)
<223>  2'Fluoro 2'deoxyribonucleotide

<220>
<221>  misc_binding
<222>  (21)..(21)
<223>  Attached to Ser(GN) via a phosphorothioate linkage

<400>  4
aacaguguuc uugcucuaua a                                                21


<210>  5
<211>  21
<212>  RNA
<213>  Artificial

<220>
<223>  Synthetic sequence


<220>
<221>  misc_binding
<222>  (1)..(3)
<223>  Phosphorothioate linkage between each ribonucleotide

<220>
<221>  misc_feature
<222>  (1)..(1)
<223>  2'Fluoro 2'deoxyribonucleotide

<220>
<221>  misc_feature
<222>  (2)..(2)
<223>  2'O Methyl ribonucleotide

<220>
<221>  misc_feature
<222>  (3)..(3)
<223>  2'Fluoro 2'deoxyribonucleotide

<220>
<221>  misc_feature
<222>  (4)..(4)
<223>  2'O Methyl ribonucleotide

<220>
<221>  misc_feature
<222>  (5)..(5)
<223>  2'Fluoro 2'deoxyribonucleotide

<220>
<221>  misc_feature
<222>  (6)..(6)
<223>  2'O Methyl ribonucleotide

<220>
<221>  misc_feature
<222>  (7)..(7)
<223>  2'Fluoro 2'deoxyribonucleotide

<220>
<221>  misc_feature
<222>  (8)..(8)
<223>  2'O Methyl ribonucleotide

<220>
<221>  misc_feature
<222>  (9)..(9)
<223>  2'Fluoro 2'deoxyribonucleotide

<220>
<221>  misc_feature
<222>  (10)..(10)
<223>  2'O Methyl ribonucleotide

<220>
<221>  misc_feature
<222>  (11)..(11)
<223>  2'Fluoro 2'deoxyribonucleotide

<220>
<221>  misc_feature
<222>  (12)..(12)
<223>  2'O Methyl ribonucleotide

<220>
<221>  misc_feature
<222>  (13)..(13)
<223>  2'Fluoro 2'deoxyribonucleotide

<220>
<221>  misc_feature
<222>  (14)..(14)
<223>  2'O Methyl ribonucleotide

<220>
<221>  misc_feature
<222>  (15)..(15)
<223>  2'Fluoro 2'deoxyribonucleotide

<220>
<221>  misc_feature

<222>  (16)..(16)
<223>  2'O Methyl ribonucleotide

<220>
<221>  misc_feature
<222>  (17)..(17)
<223>  2'Fluoro 2'deoxyribonucleotide

<220>
<221>  misc_feature
<222>  (18)..(18)
<223>  2'O Methyl ribonucleotide

<220>
<221>  misc_feature
<222>  (19)..(19)
<223>  2'Fluoro 2'deoxyribonucleotide

<220>
<221>  misc_binding
<222>  (19)..(21)
<223>  Phosphorothioate linkage between each ribonucleotide

<220>
<221>  misc_feature
<222>  (20)..(20)
<223>  2'O Methyl ribonucleotide

<220>
<221>  misc_feature
<222>  (21)..(21)
<223>  2'Fluoro 2'deoxyribonucleotide

<400>  5
aacaguguuc uugcucuaua a                                                21


<210>  6
<211>  19
<212>  RNA
<213>  Artificial

<220>
<223>  Synthetic sequence


<220>
<221>  misc_binding
<222>  (1)..(3)
<223>  Phosphorothioate linkage between each ribonucleotide

<220>
<221>  misc_feature
<222>  (1)..(1)
<223>  2'O Methyl ribonucleotide

<220>
<221>  misc_feature
<222>  (2)..(2)
<223>  2'Fluoro 2'deoxyribonucleotide

<220>
<221>  misc_feature

<222>  (3)..(3)
<223>  2'O Methyl ribonucleotide

<220>
<221>  misc_feature
<222>  (4)..(4)
<223>  2'Fluoro 2'deoxyribonucleotide

<220>
<221>  misc_feature
<222>  (5)..(5)
<223>  2'O Methyl ribonucleotide

<220>
<221>  misc_feature
<222>  (6)..(6)
<223>  2'Fluoro 2'deoxyribonucleotide

<220>
<221>  misc_feature
<222>  (7)..(7)
<223>  2'O Methyl ribonucleotide

<220>
<221>  misc_feature
<222>  (8)..(8)
<223>  2'Fluoro 2'deoxyribonucleotide

<220>
<221>  misc_feature
<222>  (9)..(9)
<223>  2'O Methyl ribonucleotide

<220>
<221>  misc_feature
<222>  (10)..(10)
<223>  2'Fluoro 2'deoxyribonucleotide

<220>
<221>  misc_feature
<222>  (11)..(11)
<223>  2'O Methyl ribonucleotide

<220>
<221>  misc_feature
<222>  (12)..(12)
<223>  2'Fluoro 2'deoxyribonucleotide

<220>
<221>  misc_feature
<222>  (13)..(13)
<223>  2'O Methyl ribonucleotide

<220>
<221>  misc_feature
<222>  (14)..(14)
<223>  2'Fluoro 2'deoxyribonucleotide

<220>
<221>  misc_feature
<222>  (15)..(15)
<223>  2'O Methyl ribonucleotide

<220>
<221>  misc_feature
<222>  (16)..(16)
<223>  2'Fluoro 2'deoxyribonucleotide

<220>
<221>  misc_feature
<222>  (17)..(17)
<223>  2'O Methyl ribonucleotide

<220>
<221>  misc_binding
<222>  (17)..(19)
<223>  Phosphorothioate linkage between each ribonucleotide

<220>
<221>  misc_feature
<222>  (18)..(18)
<223>  2'Fluoro 2'deoxyribonucleotide

<220>
<221>  misc_feature
<222>  (19)..(19)
<223>  2'O Methyl ribonucleotide

<400>  6
ucgaaguauu ccgcguacg                                                    19


<210>  7
<211>  19
<212>  RNA
<213>  Artificial

<220>
<223>  Synthetic sequence


<220>
<221>  misc_binding
<222>  (1)..(1)
<223>  Attached to [(ST23) (ps)]3 C4XLT via a phosphorothioate linkage

<220>
<221>  misc_feature
<222>  (1)..(1)
<223>  2'Fluoro 2'deoxyribonucleotide

<220>
<221>  misc_feature
<222>  (2)..(2)
<223>  2'O Methyl ribonucleotide

<220>
<221>  misc_feature
<222>  (3)..(3)
<223>  2'Fluoro 2'deoxyribonucleotide

<220>
<221>  misc_feature
<222>  (4)..(4)
<223>  2'O Methyl ribonucleotide

<220>
<221>  misc_feature
<222>  (5)..(5)
<223>  2'Fluoro 2'deoxyribonucleotide

<220>
<221>  misc_feature
<222>  (6)..(6)
<223>  2'O Methyl ribonucleotide

<220>
<221>  misc_feature
<222>  (7)..(7)
<223>  2'Fluoro 2'deoxyribonucleotide

<220>
<221>  misc_feature
<222>  (8)..(8)
<223>  2'O Methyl ribonucleotide

<220>
<221>  misc_feature
<222>  (9)..(9)
<223>  2'Fluoro 2'deoxyribonucleotide

<220>
<221>  misc_feature
<222>  (10)..(10)
<223>  2'O Methyl ribonucleotide

<220>
<221>  misc_feature
<222>  (11)..(11)
<223>  2'Fluoro 2'deoxyribonucleotide

<220>
<221>  misc_feature
<222>  (12)..(12)
<223>  2'O Methyl ribonucleotide

<220>
<221>  misc_feature
<222>  (13)..(13)
<223>  2'Fluoro 2'deoxyribonucleotide

<220>
<221>  misc_feature
<222>  (14)..(14)
<223>  2'O Methyl ribonucleotide

<220>
<221>  misc_feature
<222>  (15)..(15)
<223>  2'Fluoro 2'deoxyribonucleotide

<220>
<221>  misc_feature
<222>  (16)..(16)
<223>  2'O Methyl ribonucleotide

<220>
<221>  misc_feature
<222>  (17)..(17)

<223>  2'Fluoro 2'deoxyribonucleotide

<220>
<221>  misc_binding
<222>  (17)..(19)
<223>  Phosphorothioate linkage between each ribonucleotide

<220>
<221>  misc_feature
<222>  (18)..(18)
<223>  2'O Methyl ribonucleotide

<220>
<221>  misc_feature
<222>  (19)..(19)
<223>  2'Fluoro 2'deoxyribonucleotide

<400>  7
cguacgcgga auacuucga                                                    19


<210>  8
<211>  21
<212>  RNA
<213>  Artificial

<220>
<223>  Synthetic sequence


<220>
<221>  misc_binding
<222>  (1)..(1)
<223>  Attached to [(ST23) (ps)]3 C4XLT via a phosphorothioate linkage

<220>
<221>  misc_binding
<222>  (1)..(3)
<223>  Phosphorothioate linkage between each ribonucleotide

<220>
<221>  misc_feature
<222>  (1)..(1)
<223>  2'Fluoro 2'deoxyribonucleotide

<220>
<221>  misc_feature
<222>  (2)..(2)
<223>  2'O Methyl ribonucleotide

<220>
<221>  misc_feature
<222>  (3)..(3)
<223>  2'Fluoro 2'deoxyribonucleotide

<220>
<221>  misc_feature
<222>  (4)..(4)
<223>  2'O Methyl ribonucleotide

<220>
<221>  misc_feature
<222>  (5)..(5)

<223>  2'Fluoro 2'deoxyribonucleotide

<220>
<221>  misc_feature
<222>  (6)..(6)
<223>  2'O Methyl ribonucleotide

<220>
<221>  misc_feature
<222>  (7)..(7)
<223>  2'Fluoro 2'deoxyribonucleotide

<220>
<221>  misc_feature
<222>  (8)..(8)
<223>  2'O Methyl ribonucleotide

<220>
<221>  misc_feature
<222>  (9)..(9)
<223>  2'Fluoro 2'deoxyribonucleotide

<220>
<221>  misc_feature
<222>  (10)..(10)
<223>  2'O Methyl ribonucleotide

<220>
<221>  misc_feature
<222>  (11)..(11)
<223>  2'Fluoro 2'deoxyribonucleotide

<220>
<221>  misc_feature
<222>  (12)..(12)
<223>  2'O Methyl ribonucleotide

<220>
<221>  misc_feature
<222>  (13)..(13)
<223>  2'Fluoro 2'deoxyribonucleotide

<220>
<221>  misc_feature
<222>  (14)..(14)
<223>  2'O Methyl ribonucleotide

<220>
<221>  misc_feature
<222>  (15)..(15)
<223>  2'Fluoro 2'deoxyribonucleotide

<220>
<221>  misc_feature
<222>  (16)..(16)
<223>  2'O Methyl ribonucleotide

<220>
<221>  misc_feature
<222>  (17)..(17)
<223>  2'Fluoro 2'deoxyribonucleotide

<220>

<221>  misc_feature
<222>  (18)..(18)
<223>  2'O Methyl ribonucleotide

<220>
<221>  misc_feature
<222>  (19)..(19)
<223>  2'Fluoro 2'deoxyribonucleotide

<220>
<221>  misc_binding
<222>  (19)..(21)
<223>  Phosphorothioate linkage between each ribonucleotide

<220>
<221>  misc_feature
<222>  (20)..(20)
<223>  2'O Methyl ribonucleotide

<220>
<221>  misc_feature
<222>  (21)..(21)
<223>  2'Fluoro 2'deoxyribonucleotide

<400>  8
aacaguguuc uugcucuaua a                                                21